# EUROPEAN PATENT SPECIFICATION

(11) **EP 2 056 897 B1**
(45) Date of publication and mention of the grant of the patent: **30.12.2009**
(21) Application number: 07802809.9
(22) Date of filing: 22.08.2007
(51) Int. Cl.: A61L 31/16, A61L 31/10

(54) **MEDICAL STENT PROVIDED WITH A COMBINATION OF MELATONIN AND PACLITAXEL**
MEDIZINISCHER STENT MIT EINER KOMBINATION AUS MELATONIN UND PACLITAXEL
STENT MÉDICAL CONTENANT UNE COMBINAISON DE MÉLATONINE ET DE PACLITAXEL

(30) Priority: 23.08.2006 WO PCT/EP2006/008286
(43) Date of publication of application: 13.05.2009
(73) Proprietor: Blue Medical Devices B.V., 5708 NH Helmond (NL)
(72) Inventor: HORVERS, Ronald, Adrianus, Maria, NL-5663 PH Geldrop (NL)
(74) Representative: Brants, Johan P.E.
(86) International application number: PCT/EP2007/058743
(87) International publication number: WO 2008/023038

(56) References cited:
- WO-A-01/49268
- WO-A-2004/052401
- WO-A-2005/016400
- US-A1- 2006 083 768

## Description

### FIELD OF THE INVENTION

The present invention relates to a stent useful for expanding a vessel lumen of a subject and treating restenosis therein.

### BACKGROUND TO THE INVENTION

A stent is commonly used as a tubular structure introduced inside the lumen of a vessel to relieve an obstruction. Commonly, stents are inserted into the lumen of the vessel in a non-expanded form and are then expanded autonomously (or with the aid of a second device) *in situ.*

When a stent is used to expand a vascular lumen, restenosis (re-narrowing) may occur. Restenosis of an artherosclerotic coronary artery after a stand-alone angioplasty may occur in 10-50% of patients within 6 months, requiring either further angioplasty or coronary artery bypass graft. It is presently understood that the process of fitting a bare stent (without any drug), besides opening the artherosclerotically obstructed artery, also injures resident coronary arterial smooth muscle cells (SMC). In response to this trauma, adhering platelets, infiltrating macrophages, leukocytes, or the smooth muscle cells (SMC) themselves release cell derived growth factors with subsequent proliferation and migration of medial SMC through the internal elastic lamina to the area of the vessel intima. Further proliferation and hyperplasia of intimal SMC and, most significantly, production of large amounts of extracellular matrix over a period of 3-6 months results in the filling in and narrowing of the vascular space sufficient to significantly obstruct coronary blood flow.

To reduce or prevent restenosis, stents are provided with a means for delivering an inhibitor of SMC proliferation *i.e.* melatonin and paclitaxel directly to the wall of the expanded vessel. Such delivery means include, for example, via the struts of a stent, a stent graft, grafts, stent cover or sheath, composition with polymers (both degradable and nondegrading) to hold the drug to the stent or graft or entrapping the drug into the metal of the stent or graft body which has been modified to contain micropores or channels. Other delivery means include covalent binding of the drug to the stent via solution chemistry techniques (such as via the Carmeda process) or dry chemistry techniques (e.g. vapour deposition methods such as rf-plasma polymerization) and combinations thereof. Examples of some means for delivery are mentioned in patent document US 6,599,314.

Inhibitors of SMC proliferation include sirolimus (or rapamycin, an immunosuppressive agent) and paclitaxel (or taxol, an antiproliferative, anti-angiogenic agent). Other agents which have demonstrated the ability to reduce myointimal thickening in animal models of balloon vascular injury are heparin, angiopeptin (a somatostatin analog), calcium channel blockers, angiotensin converting enzyme inhibitors (captopril, cilazapril), cyclosporin A, trapidil (an antianginal, antiplatelet agent), terbinafine (antifungal), colchicine (antitubulin antiproliferative), and c-myc and c-myb antisense oligonucleotides.

The problem with inhibitors of the art is the delayed healing (Farb A, et al. Circulation 2001;104:473-479) and polymer-related hypertensitivity reactions. (Virmani R et al. Circulation 2004;109:r8-r42.) This increases the risk of delayed, potentially fatal thrombosis. (Virmani R et al., Liistro F, Colombo A. Heart 2001;86:262-264.)

In view of the prior art, there is a need for a new types of inhibitor of stenosis and restenosis, delivered via a stent.

### SUMMARY OF SOME EMBODIMENTS OF THE INVENTION

One embodiment of the invention is a medical stent provided with a composition comprising melatonin and paclitaxel.

Another embodiment of the invention is a medical stent as described above, wherein said stent is provided with one or more cavities configured to contain and release said composition.

Another embodiment of the invention is a medical stent as described above, wherein said stent is at least partly made from a material which is biodegradable *in situ.*

Another embodiment of the invention is a medical stent as described above, wherein said stent comprises a magnesium based alloy.

Another embodiment of the invention is a medical stent as described above, wherein said stent is at least partly made from a material which is non-biodegradable *in situ.*

Another embodiment of the invention is a medical stent as described above, wherein said stent is at least partly provided with said composition.

Another embodiment of the invention is a use of a composition comprising melatonin and paclitaxel, for the preparation of a medicament for providing a medical stent for treating smooth muscle cell, SMC, proliferation.

Another embodiment of the invention is a use as described above, wherein said stent is as defined above.

Another embodiment of the invention is a kit comprising a) at least one medical stent and b) a composition comprising melatonin and paclitaxel.

Another embodiment of the invention is a kit as described above, wherein said stent is as defined above.

Another embodiment of the invention is a medical stent, a use, or a kit as described above, wherein said composition further comprises one or more slow release agents to facilitate slow release of inhibitor.

Another embodiment of the invention is a medical stent, a use, or a kit as described above, wherein said composition further comprises one or more slow release agents to facilitate slow release of paclitaxel and melatonin.

Another embodiment of the invention is a medical stent, a use, or a kit as described above, wherein said composition further comprises one or more slow release agents to tune the slow release of the paclitaxel and melatonin.

Another embodiment of the invention is a medical stent, a use, or a kit as described above, wherein said slow release agent is any of magnesium alloys, poly(glycolic) acid, poly(lactic acid) or in general glycolic- and lactic acid based polymers, copolymers, poly caprolactones and in general, poly hydroxyl alkanoate,s poly(hydroxy alcanoic acids), Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate, poly acryl amides, poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, natural and non natural poly aminoacids , poly β-aminoesters, albumines, alginates, cellulose /cellulose acetates, chitin / chitosan, collagene, fibrine / fibrinogen, gelatine, lignine, proteine based polymers, Poly (lysine), poly (glutamate), poly (malonates), poly (hyaluronic acids), Poly nucleic acids, poly saccharides, poly (hydroxyalkanoates), poly isoprenoids, starch based polymers, copolymers thereof, linear, branched, hyperbranched, dendrimers, crosslinked, functionalised derivatives thereof, or hydrogels based on activated polyethyleneglycols combined with alkaline hydrolyzed animal or vegetal proteins.

Another embodiment of the invention is a medical stent, a use, or a kit as described above, wherein said slow release agent is a biodegradable poly (ester amide) copolymer.

Another embodiment of the invention is a medical stent, a use, or a kit as described above, wherein said melatonin is a mixture of at least one melatonin analogue optionally together with melatonin.

Another embodiment of the invention is a medical stent, a use, or a kit as described above, wherein said melatonin is a mixture of melatonin optionally together with at least one melatonin analogue.

Another embodiment of the invention is a medical stent, a use, or a kit as described above, wherein said paclitaxel is a mixture of at least one paclitaxel analogue optionally together with paclitaxel.

Another embodiment of the invention is a medical stent, a use, or a kit as described above, wherein said paclitaxel is a mixture of paclitaxel optionally together with at least one paclitaxel analogue.

Another embodiment of the invention is a medical stent, a use, or a kit as described above, wherein a melatonin analogue is any of 2-iodomelatonin, 6-chloromelatonin, 6,7-dichloro-2-methylmelatonin and 8-hydroxymelatonin.

Another embodiment of the invention is a medical stent, a use, or a kit as described above, wherein a paclitaxel analogue is a compound having formula (II), 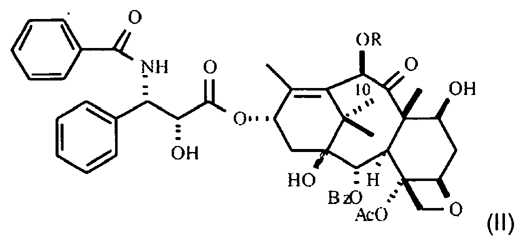 wherein R is any of Propionyl, Isobutyryl, Valeryl, Hexanoyl, Octanoyl, Decanoyl, Tridecanoyl, Methoxyacetyl, Methylthioacetyl, Methylsulfonylacetyl Acetoxyacetyl, Ethylformyl, Monosuccinyl, Crotonoyl, Acryloyl, Cyclopropanecarbonyl Cyclobutanecarbonyl, Cyclopentanecarbonyl, Cyclohexanecarbonyl, Hydrocinnamoyl, trans-Cinnamoyl, Phenylacetyl, Diphenylacetyl, Benzoyl 2-Chlorobenzoyl, 3-Chlorobenzoyl, 4-Chlorobenzoyl, 3,4-Dichlorobenzoyl, 3,5-Dichlorobenzoyl, 2,4-Dichlorobenzoyl, 3,5-Dibromobenzoyl, 4-Fluorobenzoyl, 3-Trifluoromethylbenzoyl, 4-Trifluoromethylbenzoyl, 3-Nitrobenzoyl, 4-Nitrobenzoyl, 3-Dimethylaminobenzoyl, 3-Methoxybenzoyl, 1-Naphthoyl, 2-Naphthoyl, 2-Quinolinecarbonyl, 3-Quinolinecarbonyl, 4-Quinolinecarbonyl, Indole-3-acetyl, Pyrrole-2-carbonyl, 1-Methyl-2-pyrrolecarbonyl, 2-Furoyl, 5-Bromofuroyl, 5-Nitrofuroyl, 3-Thiophenecarbonyl, 2-Thiophenecarbonyl, 2-Thiopheneacetyl, Picolinoyl, Isonicotinoyl, 5,6-Dichloronicotinoyl, 2-Methylnicotinoyl, 6-Methylnicotinoyl, 5-Bromonicotinoyl, 2-Pyrazinecarbonyl, Isobutyryl, Valeryl, Methoxyacetyl or Cyclohexanecarbonyl.

Another embodiment of the invention is a medical stent, a use, or a kit as described above, wherein the composition comprises melatonin and paclitaxel for separate or simultaneous administration.

Another embodiment of the invention is a medical stent, a use, or a kit as described above, wherein paclitaxel and melatonin are provided as separate inner and outer layers, one disposed over the other.

Another embodiment of the invention is a medical stent, a use, or a kit as described above, wherein the inner layer comprises melatonin and the outer layer comprises paclitaxel.

Another embodiment of the invention is a medical stent, a use, or a kit as described above, wherein the inner layer comprises paclitaxel and the outer layer comprises melatonin.

Another embodiment of the invention is a medical stent according, a use, or a kit as described above, wherein the inner paclitaxel layer and outer melatonin layer are devoid of slow release agents, and said stent is provided with a separate outermost layer comprising at least one slow release agent.

Another embodiment of the invention is a medical stent, a use, or a kit as described above, wherein the concentration of melatonin present on the stent is between 0.005 and 2 micrograms inclusive melatonin / mm².

Another embodiment of the invention is a medical stent, a use, or a kit as described above, wherein the concentration of paclitaxel on the stent is between 0.001 and 0.2 micrograms inclusive paclitaxel / mm².

Another embodiment of the invention is a medical stent or a kit as described above, suitable for use in inhibiting SMC proliferation.

Another embodiment of the invention is a medical stent, a use, or a kit as described above, wherein said SMC proliferation is restenosis or stenosis.

Another embodiment of the invention is a medical stent, a use, or a kit as described above, wherein the stent is placed in an artery or vein.

### FIGURE LEGENDS

**Figure 1****:** G66/11 - images from angiography. Left: after stent implantation into LAD und CX arteries. Right: control angiography after four weeks.
**Figure 2****:** G66/11 - images from LAD histology. Three samples per stent. HE and Masson Goldner staining.
**Figure 3****:** G66/12 - Images from angiography. Left: after stent implantation into LAD und CX arteries. Right: control angiography after four weeks.

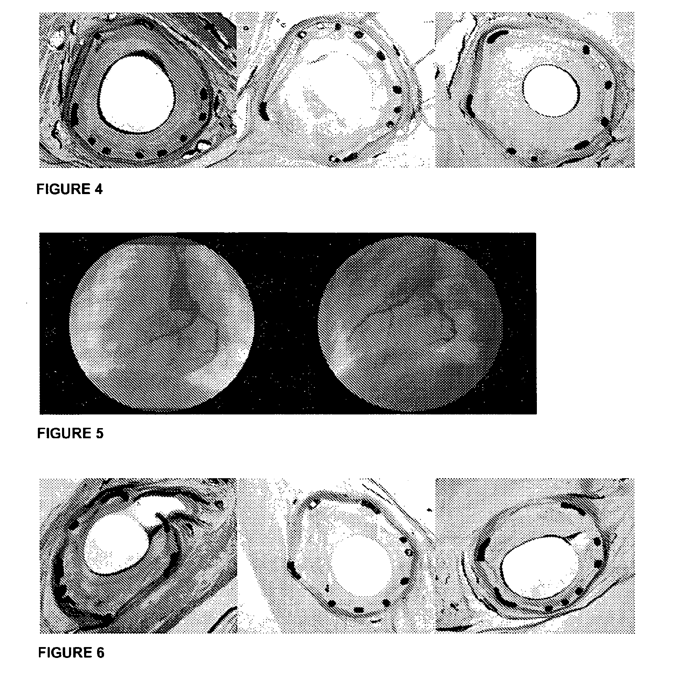

**Figure 4****:** G66/12 - Images from LAD histology. Three samples per stent. HE and Masson Goldner stain-ing.
**Figure 5****:** G66/13 - Images from angiography. Left: after stent implantation into LAD und CX arteries. Right: control angiography after four weeks.
**Figure 6****:** G66/13 - Images from LAD histology. Three samples per stent. HE and Masson Goldner staining.

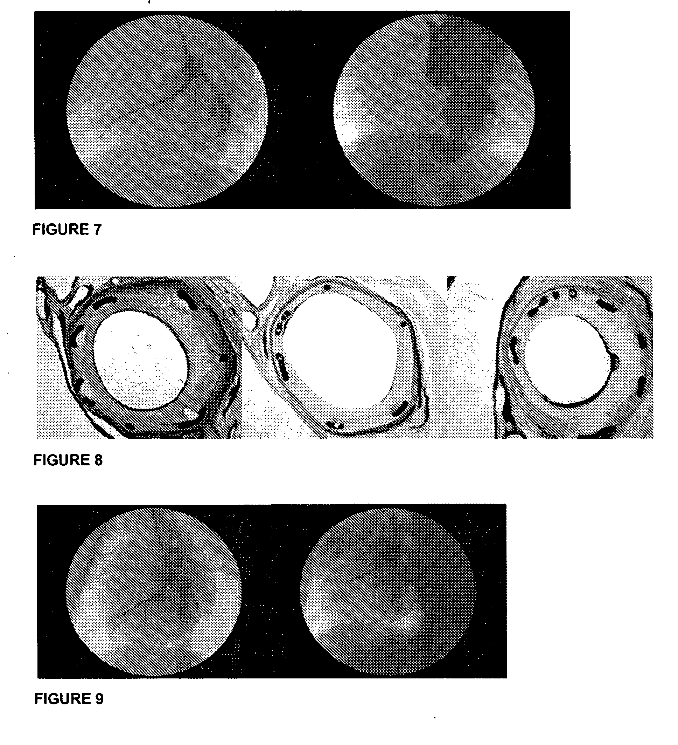

**Figure 7****:** G66/14 - Images from angiography. Left: after stent implantation into LAD und CX arteries. Right: control angiography after four weeks.
**Figure 8****:** G66/14 - Images from LAD histology. Three samples per stent. HE and Masson Goldner staining.
**Figure 9****:** G66/15 - Images from angiography. Left: after stent implantation into LAD und CX arteries. Right: control angiography after four weeks.

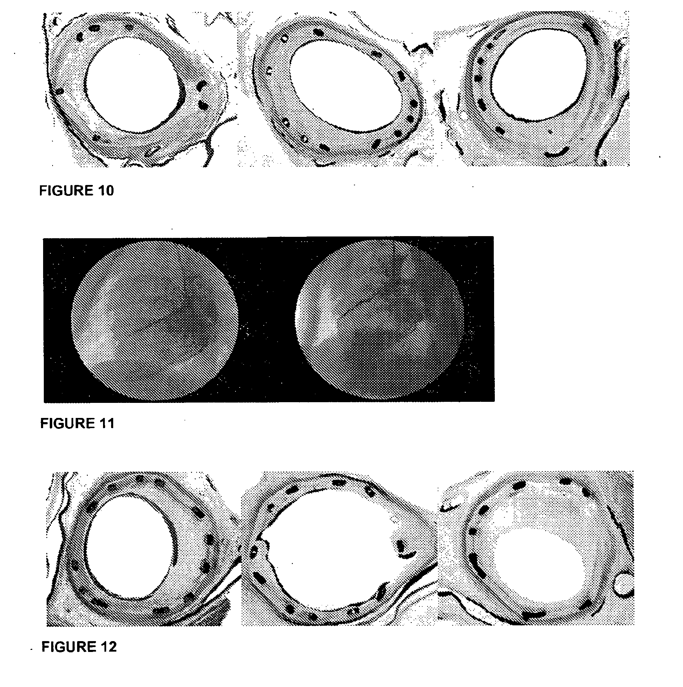

**Figure 10****:** G66/15 - Images from CX histology. Three samples per stent. HE and Masson Goldner staining.
**Figure 11****:** G66/16 - Images from angiography. Left: after stent implantation into LAD und CX arteries. Right: control angiography after four weeks.
**Figure 12****:** G66/16 - Images from CX histology. Three samples per stent. HE and Masson Goldner staining.

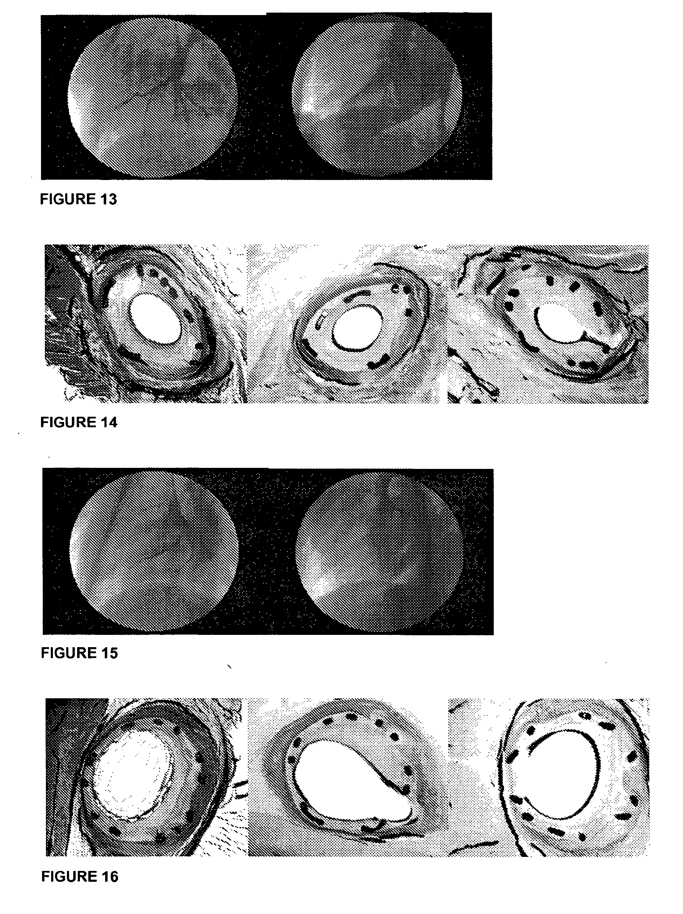

**Figure 13****:** G66/17 - Images from angiography. Left: after stent implantation into LAD und CX arteries. Right: control angiography after four weeks.
**Figure 14****:** G66/17 - Images from CX histology. Three samples per stent. HE and Masson Goldner staining.
**Figure 15****:** G66/18 - Images from angiography. Left: after stent implantation into LAD und CX arteries. Right: control angiography after four weeks.
**Figure 16****:** G66/18 - Images from CX histology. Three samples per stent. HE and Masson Goldner staining.

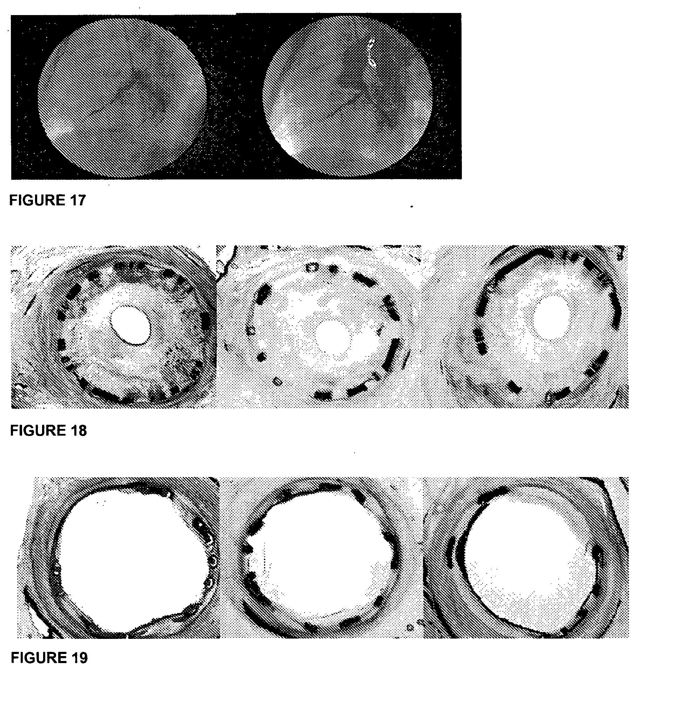

**Figure 17****:** G66/35 - Images from angiography. Left: after stent implantation into LAD und CX arteries. Right: control angiography after four weeks.
**Figure 18****:** G66/35 - Images from LAD histology. Three samples per stent. HE and Masson Goldner staining.
**Figure 19****:** G66/35 - Images from CX histology. Three samples per stent. HE and Masson Goldner staining.

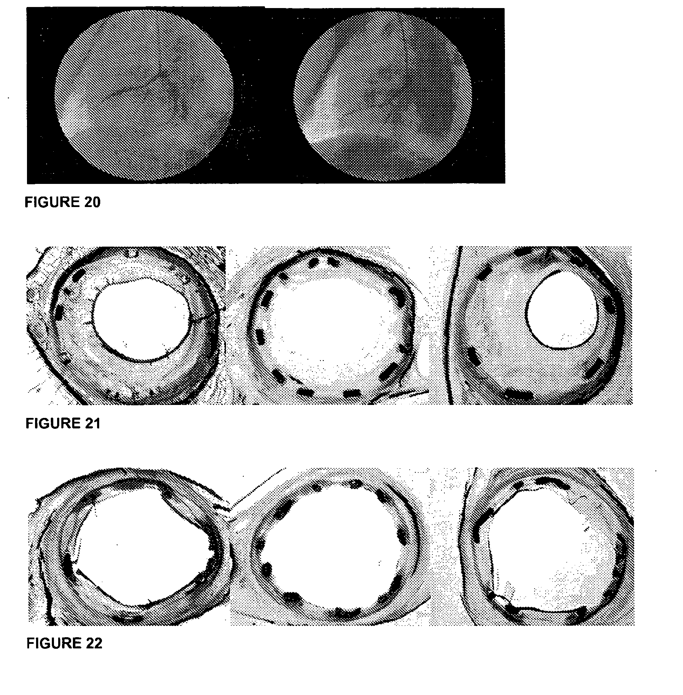

**Figure 20****:** G66/36 - Images from angiography. Left: after stent implantation into LAD und CX arteries. Right: control angiography after four weeks.
**Figure 21****:** G66/36 - Images from LAD histology. Three samples per stent. HE and Masson Goldner stain-ing.
**Figure 22****:** G66/36 - Images from CX histology. Three samples per stent. HE and Masson Goldner staining.

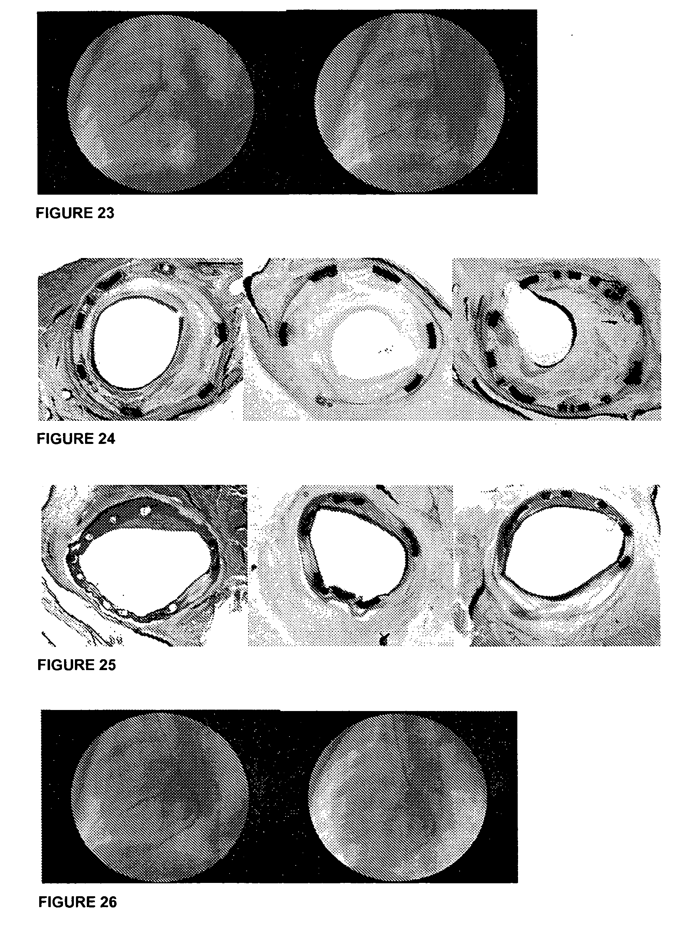

**Figure 23****:** G66/37 - Images from angiography. Left: after stent implantation into LAD und CX arteries. Right: control angiography after four weeks.
**Figure 24****:** G66/37 - Images from LAD histology. Three samples per stent. HE and Masson Goldner staining.
**Figure 25****:** G66/37 - Images from CX histology. Three samples per stent. HE and Masson Goldner staining.
**Figure 26****:** G66/39 - Images from angiography. Left: after stent implantation into LAD und CX arteries. Right: control angiography after four weeks.

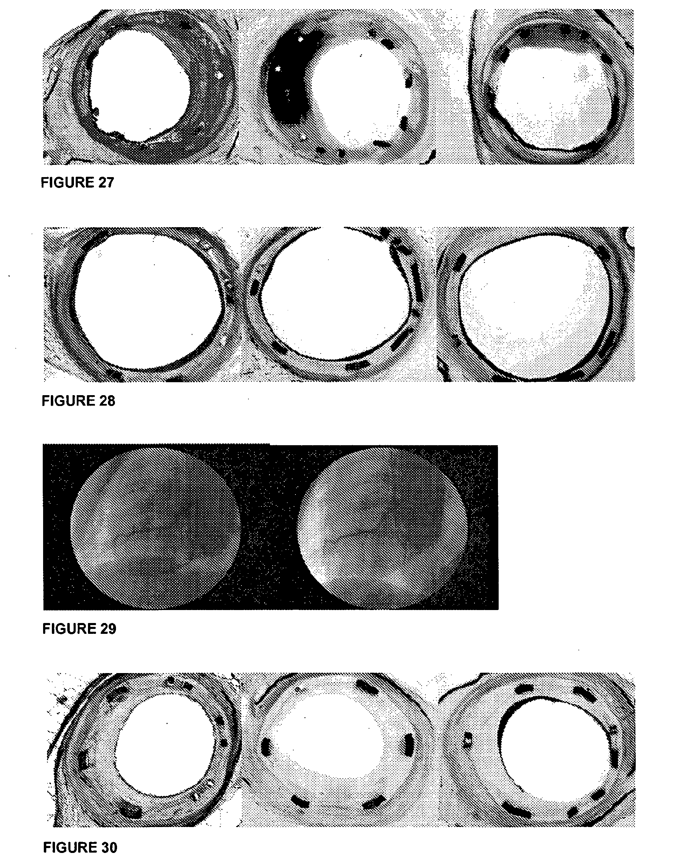

**Figure 27****:** G66/39 - Images from LAD histology. Three samples per stent. HE and Masson Goldner staining.
**Figure 28****:** G66/39 - Images from CX histology. Three samples per stent. HE and Masson Goldner staining.
**Figure 29****:** G66/40 - Images from angiography. Left: after stent implantation into LAD und CX arteries. Right: control angiography after four weeks.
**Figure 30****:** G66/40 - Images from LAD histology. Three samples per stent. HE and Masson Goldner staining.

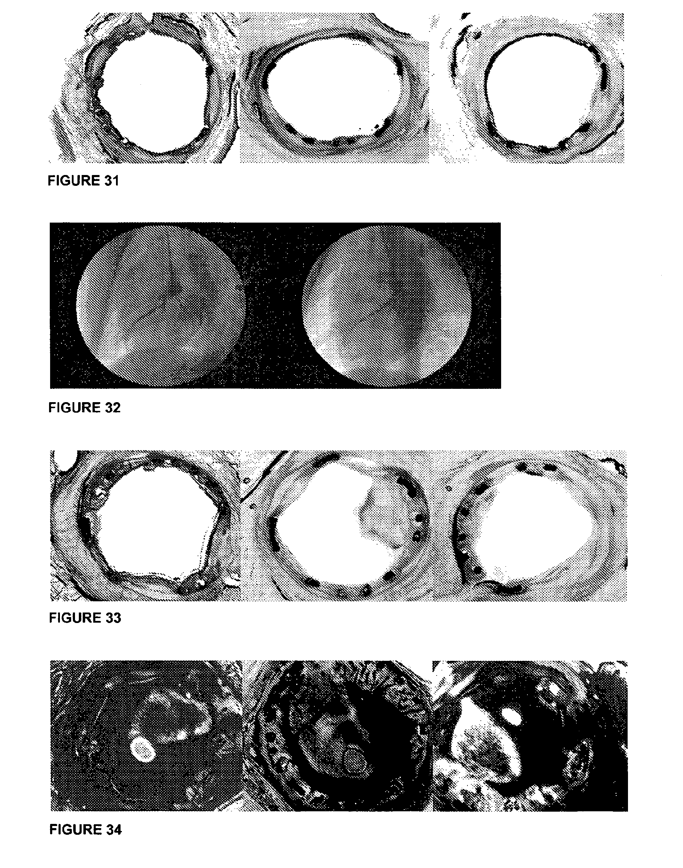

**Figure 31****:** G66/40 - Images from CX histology. Three samples per stent. HE and Masson Goldner staining.
**Figure 32****:** G66/41 - Images from angiography. Left: after stent implantation into LAD und CX arteries. Right: control angiography after four weeks.
**Figure 33****:** G66/41 - Images from LAD histology. Three samples per stent. HE and Masson Goldner staining.
**Figure 34****:** G66/41 - Images from CX histology. Three samples per stent. HE and Masson Goldner staining.

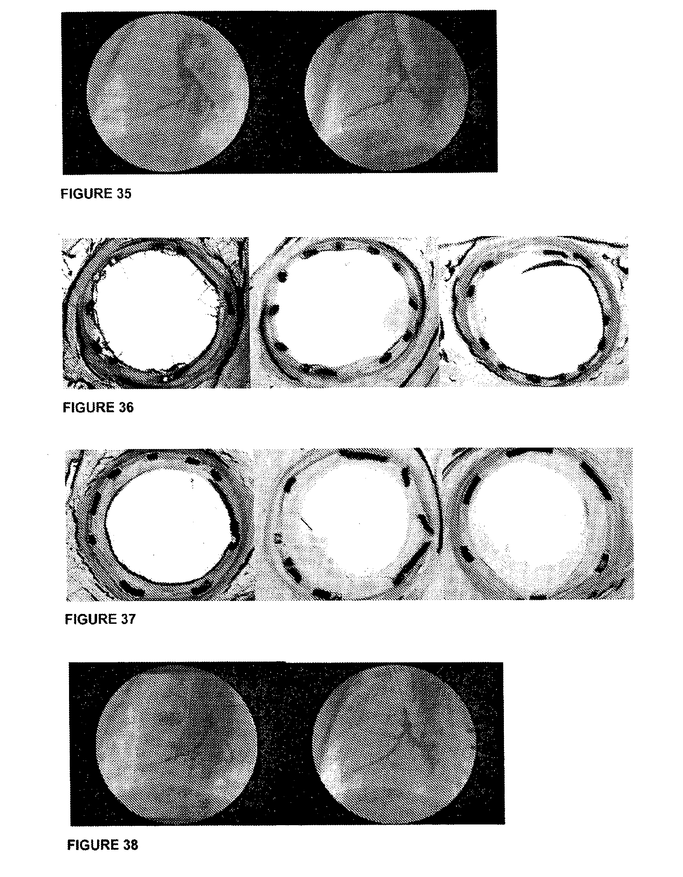

**Figure 35****:** G66/42 - Images from angiography. Left: after stent implantation into LAD und CX arteries. Right: control angiography after four weeks.
**Figure 36****:** G66/42 - Images from LAD histology. Three samples per stent. HE and Masson Goldner staining.
**Figure 37****:** G66/42 - Images from CX histology. Three samples per stent. HE and Masson Goldner staining.
**Figure 38****:** G66/43 - Images from angiography. Left: after stent implantation into LAD und CX arteries. Right: control angiography after four weeks.

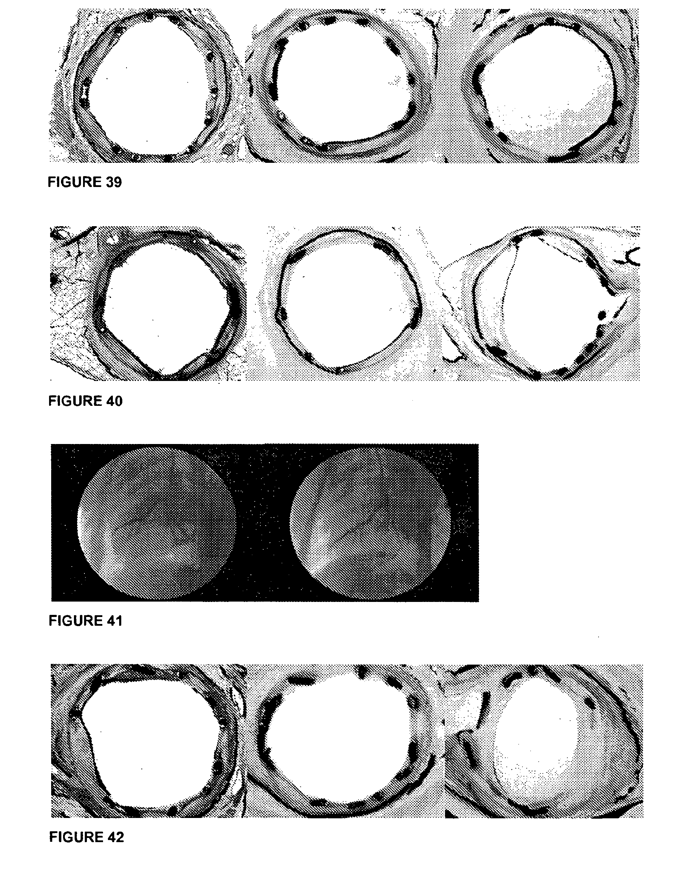

**Figure 39****:** G66/43 - Images from LAD histology. Three samples per stent. HE and Masson Goldner stain-ing.
**Figure 40****:** G66/43 - Images from CX histology. Three samples per stent. HE and Masson Goldner staining.
**Figure 41****:** G66/45 - Images from angiography. Left: after stent implantation into LAD und CX arteries. Right: control angiography after four weeks.
**Figure 42****:** G66/45 - Images from LAD histology. Three samples per stent. HE and Masson Goldner staining.

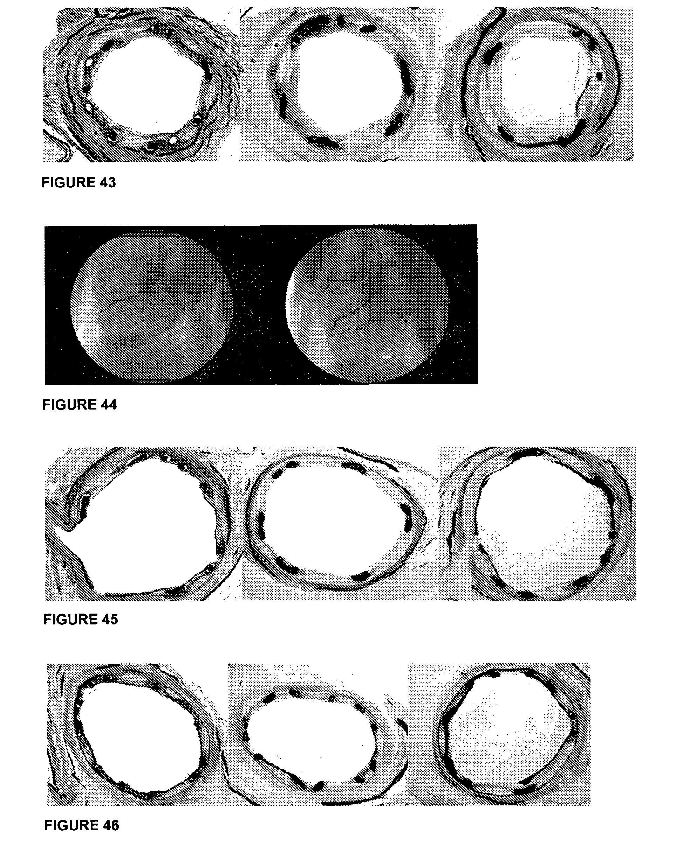

**Figure 43****:** G66/45 - Images from CX histology. Three samples per stent. HE and Masson Goldner staining.
**Figure 44****:** G66/46 - Images from angiography. Left: after stent implantation into LAD und CX arteries. Right: control angiography after four weeks.
**Figure 45****:** G66/46 - Images from LAD histology. Three samples per stent. HE and Masson Goldner staining.
**Figure 46****:** G66/46 - Images from CX histology. Three samples per stent. HE and Masson Goldner staining.

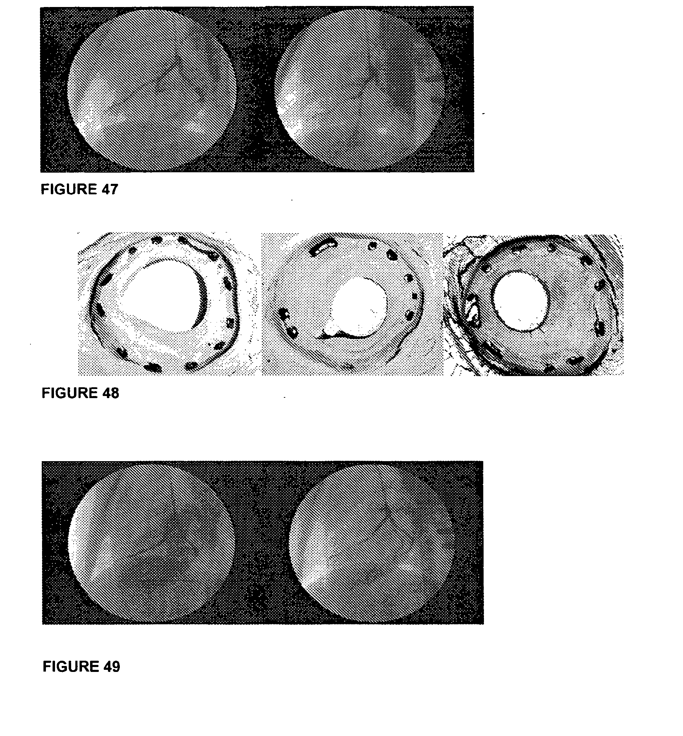

**Figure 47****:** G66/34 - Images from angiography. Left: after stent implantation into LAD und CX arteries. Right: control angiography after four weeks.
**Figure 48****:** G66/34 - Images from LAD histology. Three samples per stent. HE and Masson Goldner staining.
**Figure 49****:** G66/28 - Images from angiography. Left: after stent implantation into LAD and CX arteries. Right: control angiography after four weeks.
**Figure 50****:** G66/28 - Images from LAD histology. Three samples per stent. HE and Masson Goldner staining.
**Figure 51****:** G66/29 - Images from angiography. Left: after stent implantation into LAD und CX arteries. Right: control angiography after four weeks.
**Figure 52****:** G66/29 - Images from LAD histology. Three samples per stent. HE and Masson Goldner staining.
**Figure 53****:** G66/30 - Images from angiography. Left: after stent implantation into LAD and CX arteries. Right: control angiography after four weeks.

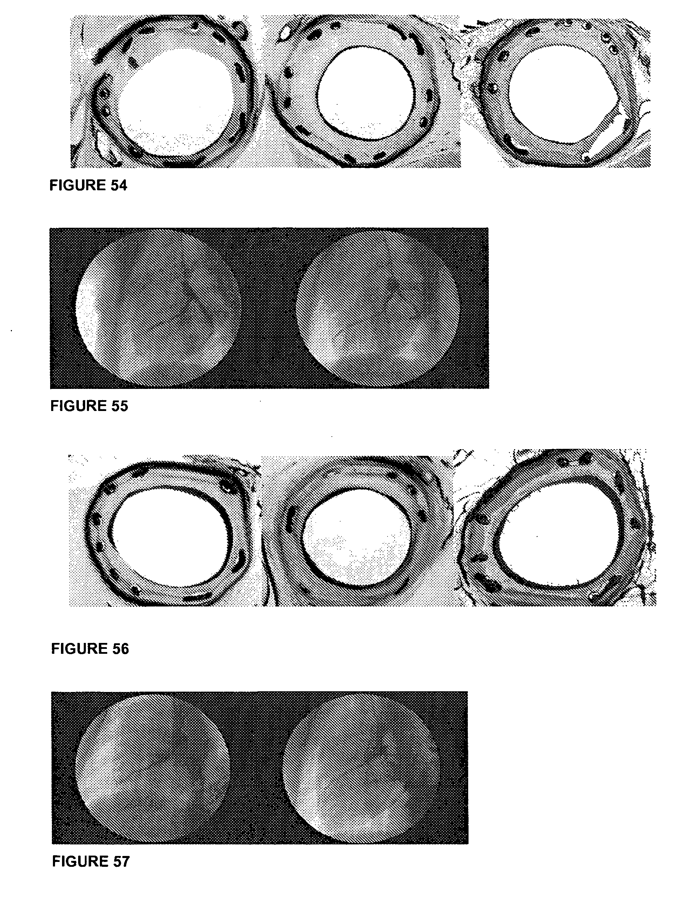

**Figure 54****:** G66/30 - Images from LAD histology. Three samples per stent. HE and Masson Goldner staining.
**Figure 55****:** G66/31 - Images from angiography. Left: after stent implantation into LAD and CX arteries. Right: control angiography after four weeks.
**Figure 56****:** G66/31 - Images from CX histology. Three samples per stent. HE and Masson Goldner staining.
**Figure 57****:** G66/32 - Images from angiography. Left: after stent implantation into LAD and CX arteries. Right: control angiography after four weeks.

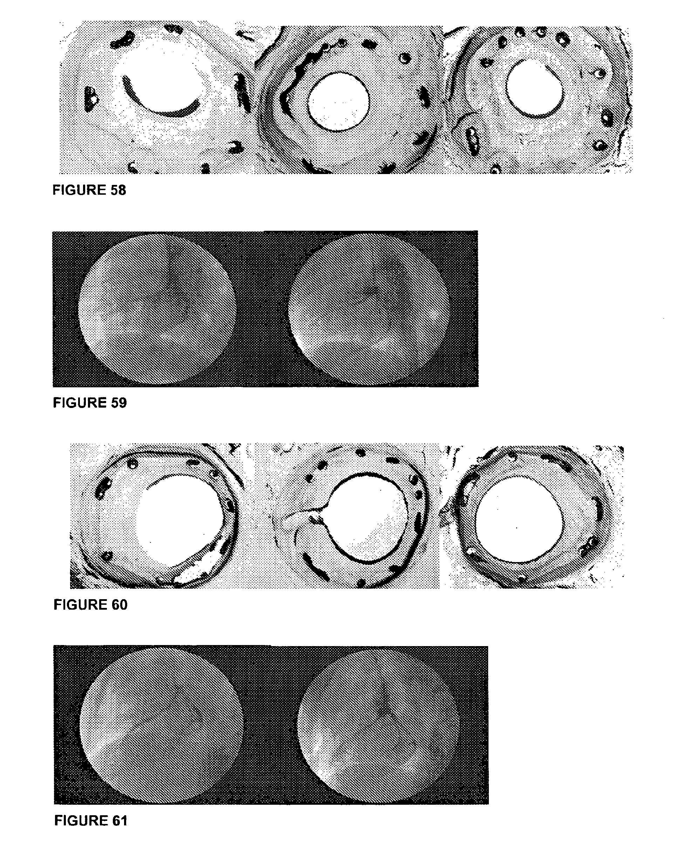

**Figure 58****:** G66/32 - Images from CX histology. Three samples per stent. HE and Masson Goldner staining.
**Figure 59****:** G66/33 - Images from angiography. Left: after stent implantation into LAD und CX arteries. Right: control angiography after four weeks.
**Figure 60****:** G66/33 - Images from CX histology. Three samples per stent. HE and Masson Goldner staining.
**Figure 61****:** G66/34 - Images from angiography. Left: after stent implantation into LAD und CX arteries. Right: control angiography after four weeks.
**Figure 62****:** G66/34 - Images from CX histology. Three samples per stent. HE and Masson Goldner staining.
**Figure 63****:** Calibration of the CellTiter 96 One solution cell proliferation assay for human coronary artery smooth cells. OD 490 nm, absorbance units recorded at 490 nm. Cells were stimulated with 10 ng/ml of PDGF-BB for 24h prior to analysis.
**Figure 64****:** 1 mM of Melatonin inhibits the proliferation of human coronary artery smooth muscle cells. Cells were stimulated with 10 ng/ml of PDGF-BB during 24h before measurement. OD 490 nm, absorbance units recorded at 490 nm. DMSO: negative control, cells were incubated with 0.1% of pure DMSO. Error bars represent the standard deviation (SD). All samples were made in triplicate.
**Figure 65****:** Melatonin up to 10 mM strongly decreased the number of human coronary artery smooth muscle cells in culture. Cells were pre-incubated with Melatonin for 120h (5 days) before measurement and were stimulated with 10 ng/ml of PDGF-BB during either 24h or 48h before measurement. OD 490 nm, absorbance units recorded at 490 nm. Error bars represent the standard deviation (SD). All samples were assessed in triplicate.
**Figure 66 (A)** and **(B)****:** Melatonin strongly inhibits the proliferation of human coronary endothelial cells in culture. M, Melatonin; OD 490 nm, absorbance units recorded at 490 nm. (A) cells were incubated 36 hours before measurement and stimulated either 24h or 48h with 10 ng/ml of VEGF-A. DMSO: negative control, cells were incubated with 1% of pure DMSO. (B) Cells were incubated for 48 hours before measurement and stimulated for 24h with VEGF-A (10 ng/ml). Error bars represent the standard deviation (SD). All samples were assessed in triplicate.
**Figure 67****:** Increasing concentrations of Melatonin do not induce any cytotoxic effects on HCAEC or on HCASMC. (A) HCAEC; (B) HCASMC. M: Melatonin; RFU, fluorescence units recorded at an excitation wavelength of 560 nm and an emission wavelength of 590 nm. Error bars represent the standard deviation (SD). All samples were assessed in triplicate.
**Figure 68****:** Melatonin inhibits [3H]-thymidine incorporation of HUVEC stimulated with VEGF-A. M: Melatonin; cpm, counts recorded for each sample using a beta-counter. Error bars represent standard deviation (SD). All samples were assessed in triplicate.
**Figure 69****:** Melatonin does not affect migration of human coronary artery endothelial cells towards VEGF-A. Human coronary artery endothelial cells were incubated for 48 hours with 2 mM of Melatonin prior to migration. Cells were allowed to migrate 4 hours (see methods) towards 10 ng/ml of VEGF-A. For Melatonin treated cells, Melatonin (2 mM) was present in both the upper and the lower chamber. Error bars represent the standard deviation of cell numbers counted in 30 power fields/grids per group.

### DETAILED DESCRIPTION OF THE INVENTION

Unless defined otherwise, all technical and scientific terms used herein have the same meaning as is commonly understood by one of skill in the art.

The articles "a" and "an" are used herein to refer to one or to more than one, *i.e.* to at least one of the grammatical object of the article. By way of example, "a stent" means one stent or more than one stent.

Throughout this application, the term "about" is used to indicate that a value includes the standard deviation of error for the device or method being employed to determine the value.

The recitation of numerical ranges by endpoints includes all integer numbers and, where appropriate, fractions subsumed within that range (e.g. 1 to 5 can include 1, 2, 3, 4 when referring to, for example, a number of stents, and can also include 1.5, 2, 2.75 and 3.80, when referring to, for example, dose concentrations). Range values are inclusive, unless otherwise stated. For example a range of 1.0 to 5.0 includes 1.0 and 5.0.

Unless otherwise stated, all quantities expressed in percent (%) are w/w.

The present invention relates to a stent provided with a composition comprising a combination of melatonin and paclitaxel for use in treating SMC proliferation in vascular vessels. Where a particular use of a composition of the present invention is described, said use may be understood as a method.

The composition can be used for treating SMC proliferation. This means that the composition can be used to treat a stenosing or restenosing cell mass. The mass may be shrunk or completely eradicated by the composition. It also means the composition can prevent restenosis when applied to regions from which stenosing or restenosing cells have been surgically removed, to reduce the possibility of regrowth. The stent allows treatment of MSC proliferation over a prolonged period.

The inventors have found for the first time that the combination of paclitaxel and melatonin acts synergistically against cellular proliferation when locally applied, particularly by way of a stent. The effect of the combination is a significant opening of a lumen narrowed by a stenosing/restenising cell mass, accompanied by reduced injury and inflammation. The effect is greater than could be expected by the mere additive effect of melatonin and paclitaxel. Therefore, administration of the combination of melatonin and paclitaxel to proliferating cells provides an effective treatment against stenosis or restenosis.

Thus, in the present invention, paclitaxel is administered to a subject in combination with melatonin, such that a synergistic anti-proliferative effect is produced. The synergistic effect refers to a greater-than-additive effect which is produced by a combination of two drugs, and which exceeds that which would otherwise result from individual administration of either drug alone. Administration of paclitaxel in combination with melatonin unexpectedly results in a synergistic effect by providing greater efficacy than would result from use of either of the agents alone. Melatonin enhances paclitaxel's effects. Therefore, lower doses of one or both of the agents may be used in treating stenosis or restenosis, resulting in increased therapeutic efficacy and decreased side-effects.

One measure of synergy between two compounds is the combination index (Cl) method of Chou and Talalay [Chou and Talalay, Quantitative analysis of dose-effect relationships: the combined effects of multiple drugs or enzyme inhibitors. Adv. Enzyme Regul., 22:27-55, 1984.], which is based on the median-effect principle. This method calculates the degree of synergy, additivity, or antagonism between two drugs at various levels of cytotoxicity. Where the Cl value is less than 1, there is synergy between the two compounds. Where the Cl value is 1, there is an additive effect, but no synergistic effect. Cl values greater than 1 indicate antagonism. The smaller the Cl value, the greater the synergistic effect. Another measurement of synergy is the fractional inhibitory concentration (FIC) [Hall et al., The fractional inhibitory concentration (FIC) index as a measure of synergy. J. Antimicrob. Chemother., 11(5):427-33, 1983.]. This fractional value is determined by expressing the IC50 of a drug acting in combination, as a function of the IC50 of the drug acting alone. For two interacting drugs, the sum of the FIC value for each drug represents the measure of synergistic interaction. Where the FIC is less than 1, there is synergy between the two drugs. An FIC value of 1 indicates an additive effect. The smaller the FIC value, the greater the synergistic interaction. In the method of the present invention, combination therapy using paclitaxel and melatonin preferably results in an antineoplastic effect that is greater than additive, as determined by any of the measures of synergy known in the art.

The term "vessel" as used herein refers to a fluid-carrying duct of a subject suitable for placing a medical stent therein. Such a vessel may be narrowed by a medical condition such as stenosis or atherosclerosis. Examples of vessels include, but are not limited to arteries and veins.

A "subject" according to the present invention may be any living body susceptible to treatment by a stent. Examples include, but are not limited to humans, dogs, cats, horses, cows, sheep, rabbits, and goats etc.

Where a stent is provided with a composition, it means the composition is deposited on, or within the stent, so the composition can be released when the stent contacts the vessel inner wall. Such stents include drug-releasing stents. The stent may be coated with the composition, Alternatively, the stent may be impregnated with composition, Alternatively, the stent may comprise cavities in which the composition resides. Various embodiments of the stent are described below.

A composition as used herein may comprise at least the combination of melatonin and paclitaxel. In the preferred mode of the invention, a stent is provided with the combination of melatonin and paclitaxel, together with a slow release polymer or agent.

The composition may be provided on the stent such that the melatonin is separately or simultaneously administered with respect to the paclitaxel.

By simultaneous administration it is meant the melatonin and paclitaxel are administered to a subject at the same time. For example, stent may be provided with a composition comprising a mixture melatonin and paclitaxel, optionally together with a slow release polymer or agent.

By separate administration it is meant that the melatonin and paclitaxel are separately administered to a subject at the same time or substantially the same time. The components are present on the stent as separate, unmixed preparations. For example, stent may be provided with a layer comprising paclitaxel and a separate layer comprising melatonin. The paclitaxel and melatonin respective layers do not intermix on the stent prior to administration, thereby giving rise to separately administered compounds.

The maximum thickness of a separately administered paclitaxel layer may be 0.07 mm, 0.08 mm, 0.09 mm, 0.1 mm, 0.12 mm, 0.14 mm, 0.16 mm, 0.18 mm, 0.2 mm, 0.22 mm, 0.24 mm, 0.26 mm, 0.28 mm, 0.3 mm, 0.32 mm, 0.34 mm or 0.36 mm, or a value in the range between any two of the aforementioned values, preferably between 0.07 to 0.36 mm, more preferably between 0.1 to 0.2 mm.

The maximum thickness of a separately administered melatonin layer may be 0.07 mm, 0.08 mm, 0.09 mm, 0.1 mm, 0.12 mm, 0.14 mm, 0.16 mm, 0.18 mm, 0.2 mm, 0.22 mm, 0.24 mm, 0.26 mm, 0.28 mm, 0.3 mm, 0.32 mm, 0.34 mm or 0.36 mm, or a value in the range between any two of the aforementioned values, preferably between 0.07 to 0.36 mm, more preferably between 0.1 to 0.2 mm.

The maximum thickness of a separately administered polymer layer may be 0.07 mm, 0.08 mm, 0.09 mm, 0.1 mm, 0.12 mm, 0.14 mm, 0.16 mm, 0.18 mm, 0.2 mm, 0.22 mm, 0.24 mm, 0.26 mm, 0.28 mm, 0.3 mm, 0.32 mm, 0.34 mm or 0.36 mm, or a value in the range between any two of the aforementioned values, preferably between 0.07 to 0.36 mm, more preferably between 0.1 to 0.2 mm.

According to one aspect of the invention, the separate layers of paclitaxel and melatonin are disposed one over the other, so that an outer layer is first administered followed by an inner layer *in situ.* "Inner layer" and "outer layer" refer to the relative positions of the layers, one over the other, with respect to the stent. The inner layer is the layer of the two, closer to the stent, and may or may not be the innermost layer. For example, the stent may first be coated with an innermost layer of priming substance to which the inner layer can bind. The outer layer is positioned at least partially over the inner layer. It is the layer of the two layers, further from the bare stent surface; it will be the layer of the two layers, initially closer to the site of administration. The outer layer may or may not be the outermost layer. For example, the stent may be further coated with a layer of polymer (e.g. slow release polymer) or other additional consecutive separate layers of paclitaxel and melatonin for example whereby the final coating forms the outermost layer. One or more intervening layers may be present between the inner and outer layers. According to one aspect of the invention, one or more intervening layers comprises polymer (e.g. slow release polymer). Alternatively, no intervening layers may be present between the inner and outer layers.

According to one aspect of the invention, an inner layer comprises paclitaxel and an outer layer comprises melatonin. In a more preferred embodiment, an inner layer comprises paclitaxel devoid of any polymer (e.g. devoid of slow release polymer) and an outer layer comprises melatonin. In a more preferred embodiment, an inner layer comprises paclitaxel devoid of any polymer (e.g. devoid of slow release polymer), and an outer layer comprises melatonin and slow release polymer. In a most preferred embodiment, an inner layer comprises paclitaxel devoid of any polymer (e.g. devoid of slow release polymer), an outer layer comprises melatonin devoid of any polymer (e.g. devoid of slow release polymer), and outermost layer comprises slow release polymer. The layers are distinct, separate and do not intermix in the undeployed stent.

Such stents are prepared by applying a first coating (e.g. paclitaxel devoid of polymer), allow the coating to dry, and applying a second coating (e.g. melatonin devoid of polymer) and allowing the second coating to dry. Subsequent layers (e.g. slow release polymer) are applied using similar steps of application and drying to ensure the respective coatings do not mix.

According to one aspect of the invention, the separate layers of paclitaxel and melatonin are provided at different locations on the stent thereby achieving physical separation. According to one embodiment of the invention, a stent is provided with a plurality of discrete depositions of paclitaxel and a plurality of discrete depositions of melatonin, wherein the respective depositions do not overlap. Preferably the number of respective discrete depositions is equal for a given area of stent.

According to one embodiment of the invention, a stent may be provided with a plurality of deposited paclitaxel-comprising strips, interlaced with a plurality of similarly deposited melatonin-comprising strips, which strips do not overlap. The strips may be aligned along the longitudinal axis of the stent, perpendicular to the longitudinal axis of the stent or at an angle thereto. The ratio of melatonin:paclitaxel strips may be 0.80, 0.85, 0.90, 0.95, 1.0, 1.05, 1.10 or 1.15, 1.2:1 or a value in the range between any two of the aforementioned values, preferably 0.80 to 1.20:1.

According to one embodiment, a stent of the invention may be provided with a plurality of paclitaxel-comprising spots wherein at least part of a space between the spots is provided with a melatonin-comprising spot, where the respective spots do not overlap. The ratio of melatonin:paclitaxel spots may be 0.80, 0.85, 0.90, 0.95, 1.0, 1.05, 1.10 or 1.15, 1.2:1 or a value in the range between any two of the aforementioned values, preferably 0.80 to 1.20:1.

In a preferred aspect, one set of discrete depositions comprises paclitaxel and another set of discrete depositions melatonin. In a more preferred embodiment, one set of discrete depositions comprises paclitaxel devoid of any polymer (e.g. slow release polymer) and another set of discrete depositions comprises melatonin. In a most preferred embodiment, one set of discrete depositions comprises paclitaxel devoid of any polymer (e.g. slow release polymer) and another set of discrete depositions comprises melatonin, and a distinct and separate layer of polymer (e.g slow release) is provided covering both set of spots.

The medical uses of the composition described below, also apply to the composition comprising melatonin and paclitaxel, for simultaneous, separate or sequential administration to a subject as disclosed here above.

A composition of the invention may comprise additional substances, such as, for example, those that facilitate sobulisation of the melatonin and paclitaxel and/or the attachment of the melatonin and paclitaxel to the stent, those that release the melatonin and paclitaxel in a controlled manner *in situ,* and those that facilitate the functioning or the performance of the stent *in situ.* Such additional substances are known to the skilled artisan.

### Stents

Stents according to the invention may be any stent that is capable of being provided with a composition according to the invention. Stents have been extensively described in the art. For example they may be cylinders which are perforated with passages that are slots, ovoid, circular, regular, irregular or the like shape. They may also be composed of helically wound or serpentine wire structures in which the spaces between the wires form the passages. Stents may also be flat perforated structures that are subsequently rolled to form tubular structures or cylindrical structures that are woven, wrapped, drilled, etched or cut to form passages. A stent may also be combined with a graft to form a composite medical device, often referred to as a stent graft. A stent should capable of being coated with a composition described herein.

Stents may be made of biocompatible materials including biostable and bioabsorbable materials. Suitable biocompatible metals include, but are not limited to, stainless steel, tantalum, titanium alloys (including nitinol), and cobalt alloys (including cobalt-chromium-nickel alloys). Stents may be made of biocompatible and bioabsorbable materials such as magnesium based alloys. Bioabsorbable stents may inserted at the site of treatment, and left in place. The structure of the stent does not become incorporated into the wall of the vessel being treated, but is degraded with time. Where the stent is made from biostable (non-absorbable) materials, the stent may be inserted for the duration of treatment and later removed.

Suitable nonmetallic biocompatible materials include, but are not limited to, polyamides, polyolefins *(i.e.* polypropylene, polyethylene etc.), nonabsorbable polyesters (i.e. polyethylene terephthalate), and bioabsorbable aliphatic polyesters (i.e. homopolymers and copolymers of lactic acid, glycolic acid, lactide, glycolide, para-dioxanone, trimethylene carbonate, epsilon -caprolactone, etc. and blends thereof), lactide capronolactone, poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D, L-lactide-co-glycolide) (PLA/PGA), poly(glycolide-co-trimethylene carbonate) (PGA/PTMC), polyethylene oxide (PEO), polydioxanone (PDS), polycaprolactone (PCL), polyhydroxylbutyrate (PHBT), poly(phosphazene), polyD,L-lactide-co-caprolactone) (PLA/PCL), poly(glycolide-co-caprolactone) (PGA/PCL), polyanhydrides (PAN), poly(ortho esters), poly(phoshate ester), poly(amino acid), poly(hydroxy butyrate), polyacrylate, polyacrylamid, poly(hydroxyethyl methacrylate), elastin polypeptide co-polymer, polyurethane, starch, polysiloxane and their copolymers.

Stents according to the present invention can be of any type known in the art suitable for delivery of the combination of paclitaxel and melatonin. As such these stents can be balloon expandable, self-expanding, provided with cavities etched into the framework of the stent for containing substances, stents provided with means for containing substances, bioabsorbable stents. The stent may also be made from different sorts of wires, for instance from polymeric biodegradable wires containing the active compound, interweaved with the metallic struts of the stent (balloon expendable or self-expandable stent).

Self expanding stents may be braided, from flexible metal, such as special alloys, from nitenol, from phynox. Self-expandable stents made from nitenol may be laser cut. One or more of the filaments that compose the self-expandable stent can be made from a polymer or a tube that elutes the anti-energetic compound.

Variations of stent and polymers are described in more detailed below.

Examples of stents include, but are not limited to, those described in US 4,733,665, US 4,800,882, US 4,886,062, US 5,514,154, US 6,398,806, EP 1 140 242, US 6,248,129, EP 1 217 969, EP 1 359 868, EP 1 349 517, EP 1 347 717, EP 1 318 765, EP 1 296 615, EP 1 229 864, EP 1 194 081, EP 1 191 904, EP 1 139 914, EP 1 087 701, EP 1 079 768, EP 1 018 985, EP 0 749 729, EP 0 556 850, EP 1 328 212, EP 1 322 256, EP 0 740 558, EP 1 251 800, EP 1 251 799, EP 1 235 856, EP 1 227 772, EP 1 123 065, EP 1 112 040, EP 1 094 764, EP 1 076 534, EP 1 065 993, EP 1 059 896, EP 1 059 894, EP 1 049 421, EP 1 027 012, EP 1 001 718, EP 0 986 416, EP 0 859 644, EP 0 740 558, EP 0 664 689, EP 0 556 850, EP 1 372 535, US 6,669,723, US 6,663,660, EP 1 065 996, US 6,652,577, US 6,652,575, EP 1 360 943, US 6,620,202, US 6,610,087, US 6,602,283, US 6,592,617, US 6, 585,758, US 6,585,753, EP 1 318 771, EP 1 318 768, US 6,579,308, US 2003/0109931, EP 1 163 889, EP 0 790 811, US 6,551,351, US 6,540,777, US 6,533,810, US 6,530,950, US 6,527,802, US 6,524,334, US 6,506,211, US 6,488,703, US 6,485,590, US 6,478,816, US 6,478,815, US 6,475,233, EP 1 251 891, US 6,471,720, US 6,428,569, EP 1 223 873, EP 0 758 216, US 6,416,543, US 6,641,538, EP 1 217 101, US 6,409,754, US 6,409,753, US 2002/0077592, EP 0 754 017, US 6,398,807, EP 1 207 815, EP 0 775 471, US 6,395,020, IS 6,391,052, US 6,387,122, US 6,379,379, US 6,355,070, US 6,348,065, EP 1 173 110, US 6,334,870, EP 1 163 889, IS 6,325,822, US 6,319,277, EP 1 144 042, EP 0 622 059, US 6,261,319, EP 1 112 039, US 6,251,134, US 6,240,978, US 6,217,607, US 6,193,744, US 6,174,328, EP 1 065 996, US 6,168,621, US 6,168,619, US 6,159,238, US 6,159,237, US 6,146,416, US 6,143,002, EP 0 986 416, EP 1 032 329, EP 1 019 107, EP 1 011 529, EP 1 011 528, US 6,071,308, EP 0 859 644, US 6,059,810, US 6,042,597, US 6,033,433, EP 0 979 059, US 6,022,371, US 5,993,483, US 6,957,974, US 5,594,744, WO 99/44535, EP 0 934 034, EP 0 934 033, US 5,922,019, WO 99/16388, EP 0 858 298, US 5,891,191, US 5,888,201, US 5,876,448, US 5,876,445, US 5,868,781, US 5,855,600, WO 98/55174, EP 0 746 375, US 5,824,045, US 5,800,511, EP 0 836 839, EP 0 767 685, US 5,683,488, EP 1 212 987, EP 1 212 987, EP 1 151 730, EP 1 151 730, EP 0 722 701, EP 1 236 447, EP 1 293 178, EP 1 236 449, EP 1 190 685, EP 1 138 280, EP 1 346 706, EP 1 330 993, EP 1 258 231, EP 1 325 717, EP 1 302 179, EP 1 095 634, EP 1 302 179, EP 1 295 615, EP 1 293 178, EP 1 266 638, EP 1 266 638, EP 1 260 197, EP 1 236 448, EP 1 236 446, EP 1 236 445, EP 1 258 231, EP 1 236 449, EP 1 236 448, EP 1 236 447, EP 1 236 446, EP 1 236 445, EP 1 112 724, EP 1 190 685, EP 1 179 323, EP 1 138 280, EP 1 112 724, EP 1 031 330, EP 0 937 442, EP 1 042 997, EP 1 031 330, EP 1 025 812, EP 1 000 590, EP 0 950 386, EP 0 873 734, EP 0 937 442, EP 0 864 302, EP 0 928 606, EP 0 864 302, EP 0 806 191, EP 1 212 988, EP 1 266 640, EP 1 266 639, EP 0 879 027, EP 1 226 798, US 6,656,215, EP 1 362 564, EP 0 904 009, EP 1 212 990, EP 1 155 664, EP 1 121 911, EP 1 266 640, EP 1 266 639, EP 1 031 329, EP 1 212 990, EP 1 212 988, EP 0 698 380, EP 1 155 664, EP 1 121 911, US 6,270,521, EP 1 031 329, EP 0 928 605, EP 0 928 605, EP 0 904 009, EP 0 903 123, EP 0 879 027, US 5,840,009, EP 0 839 506, US 5,741,324, EP 0 823 245, EP 0 698 380, EP 0 606 165, US 6,626,938, US 6,613,075, US 6,565,600, US 6,562,067, US 6,547,817, US 6,540,775, US 6,520,985, US 6,494,908, US 6,482,227, US 6,423,091, US 6,342,067, US 6,325,825, US 6,315,708, US 6,267,783, US 6,267,777, US 6,264,687, US 6,258,116, US 6,238,409, US 6,214,036, US 6,190,406, US 6,176,872, US 6,162,243, US 6,129,755, US 6,053,873, EP 0 904 009, US 6,019,778, EP 0 974 315, US 6,017,363, EP 0 951 877, EP 0 970 711, EP 0 785 807, US 6,013,019, EP 0 947 180, US 5,997,570, US 5,980,553, EP 0 951 877, US 5,938,682, US 5,895,406, US 5,891,108, US 5,882,335, US 5,792,172, US 5,782,906, EP 0 810 845, US 5,695,516, US 5,674,241, US 5,609,605, US 5,607,442, US 5,599,291, US 5,135,536, US 5,116,365, EP 0 378 151, US 4,994,071, EP 0 378 151, US 4,856,516.

### Polymers

It is an aspect of the invention that the stent is provided with a combination of paclitaxel and melatonin by way of at least partially coating the stent with a composition comprising a polymer, paclitaxel and melatonin. A polymer according to the present invention is any that facilitates attachment of the paclitaxel and melatonin to the stent (*i.e*. stent and/or membrane) and/or facilitates the controlled release of paclitaxel and melatonin. Preferably, the polymer is used to tune the slow release of paclitaxel and melatonin to obtain an optimal response; in words, to reduce the rate at which the paclitaxel and melatonin are released from the stent. A too fast release of the composition, particularly of paclitaxel could result in vessel wall toxicity. By tuning the release, and the vessel wall is impregnated for a certain time period (e.g. 2-6 weeks) with the composition resulting in an optimal effect on the healing response. The skilled person will understand that the type of polymer, concentration, thickness, and the use of multiple layers in a sequential release (described below) can be adjusted within the routine practices of the skilled person to obtain an optimum release.
Polymers suitable for use in the present invention are any that are capable of attaching to the stent and releasing paclitaxel and melatonin. They must be biocompatible to minimize irritation to the vessel wall. Polymers may be, for example, film-forming polymers that are absorbable or non-absorbable. The polymer may be biostable or bioabsorbable depending on the desired rate of release or the desired degree of polymer stability.

Suitable bioabsorbable polymers that could be used include polymers selected from the group consisting of aliphatic polyesters, poly(amino acids), copoly(ether-esters), polyalkylenes oxalates, polyamides, poly(iminocarbonates), polyanhydrides, polyorthoesters, polyoxaesters, polyamidoesters, polylactic acid (PLA), polyethylene oxide (PEO), polycaprolactone (PCL), polyhydroxybutyrate valerates, polyoxaesters containing amido groups, poly(anhydrides), polyphosphazenes, silicones, hydrogels, biomolecules and blends thereof. Another polymer is any poly(ester amide).

For the purpose of the present invention, aliphatic polyesters include homopolymers and copolymers of lactide (which includes lactic acid D-, L- and meso lactide), epsilon - caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, 6,6-dimethyl-1,4-dioxan-2-one and polymer blends thereof. Poly(iminocarbonate) for the purpose of this invention include as described by Kemnitzer and Kohn, in the Handbook of Biodegradable Polymers, edited by Domb, Kost and Wisemen, Hardwood Academic Press, 1997, pages 251-272. Copoly(ether-esters) for the purpose of this invention include those copolyester-ethers described in Journal of Biomaterials Research, Vol. 22, pages 993-1009, 1988 by Cohn and Younes and Cohn, Polymer Preprints (ACS Division of Polymer Chemistry) Vol. 30(1), page 498, 1989 (e.g. PEO/PLA). Polyalkylene oxalates for the purpose of this invention include Patent Nos. 4,208,511; 4,141,087; 4,130,639; 4,140,678; 4,105,034; and 4,205,399.

Polyphosphazenes, co-, ter- and higher order mixed monomer based polymers made from L-lactide, D,L-lactide, lactic acid, glycolide, glycolic acid, para-dioxanone, trimethylene carbonate and epsilon -caprolactone such as are described by Allcock in The Encyclopedia of Polymer Science, Vol. 13, pages 31-41, Wiley Intersciences, John Wiley & Sons, 1988 and by Vandorpe, Schacht, Dejardin and Lemmouchi in the Handbook of Biodegradable Polymers, edited by Domb, Kost and Wisemen, Hardwood Academic Press, 1997, pages 161-182

Polyanhydrides from diacids of the form HOOC-C₆H₄-O-(CH₂)ₘ-O-C₆H₄-COOH wherein m is an integer in the range of from 1 to 11, 3 to 9, 3 to 7, 2 to 6 or preferably 2 to 8, and copolymers thereof with aliphatic alpha-omega diacids of up to 8, 9, 10, 11 or preferably 12 carbons. Polyoxaesters polyoxaamides and polyoxaesters containing amines and/or amido groups are described in one or more of the following U.S. Patent Nos. 5,464,929; 5,595,751; 5,597,579; 5,607,687; 5,618,552; 5,620,698; 5,645,850; 5,648,088; 5,698,213 and 5,700,583; (which are incorporated herein by reference). Polyorthoesters such as those described by Heller in Handbook of Biodegradable Polymers, edited by Domb, Kost and Wisemen, Hardwood Academic Press, 1997, pages 99-118.

Other polymeric biomolecules for the purpose of this invention include naturally occurring materials that may be enzymatically degraded in the human body or are hydrolytically unstable in the human body such as fibrin, fibrinogen, collagen, gelatin, glycosaminoglycans, elastin, and absorbable biocompatible polysaccharides such as chitosan, starch, fatty acids (and esters thereof), glucoso-glycans and hyaluronic acid.

Suitable biostable polymers with relatively low chronic tissue response, such as polyurethanes, silicones, poly(meth)acrylates, polyesters, polyalkyl oxides (polyethylene oxide), polyvinyl alcohols, polyethylene glycols and polyvinyl pyrrolidone, as well as, hydrogels such as those formed from crosslinked polyvinyl pyrrolidinone and polyesters could also be used. Other polymers could also be used if they can be dissolved, cured or polymerized on the stent. These include polyolefins, polyisobutylene and ethylene-alphaolefin copolymers; acrylic polymers (including methacrylate) and copolymers, vinyl halide polymers and copolymers, such as polyvinyl chloride; polyvinyl ethers, such as polyvinyl methyl ether; polyvinylidene halides such as polyvinylidene fluoride and polyvinylidene chloride; polyacrylonitrile, polyvinyl ketones; polyvinyl aromatics such as polystyrene; polyvinyl esters such as polyvinyl acetate; copolymers of vinyl monomers with each other and olefins, such as etheylene- methyl methacrylate copolymers, acrylonitrile-styrene copolymers, ABS resins and ethylene-vinyl acetate copolymers; polyamides,such as Nylon 66 and polycaprolactam; alkyd resins; polycarbonates; polyoxymethylenes; polyimides; polyethers; epoxy resins, polyurethanes; rayon; rayon-triacetate, cellulose, cellulose acetate, cellulose acetate butyrate; cellophane; cellulose nitrate; cellulose propionate; cellulose ethers (i.e. carboxymethyl cellulose and hydoxyalkyl celluloses); and combinations thereof. Polyamides for the purpose of this application would also include polyamides of the form-NH-(CH₂)n-CO- and NH-(CH₂)x-NH-CO-(CH₂)y-CO, wherein
n is an integer in from 5 to 15, 7 to 11, 8 to 10 or preferably 6 to 13;
x is an integer in the range of from 5 to 14, 7 to 11, 8 to 10 or preferably 6 to 12; and
y is an integer in the range of from 3 to 18, 5 to 14, 6 to 10 or preferably 4 to 16. The list provided above is illustrative but not limiting.

Other polymers suitable for use in the present invention are bioabsorbable elastomers, more preferably aliphatic polyester elastomers. In the proper proportions aliphatic polyester copolymers are elastomers. Elastomers present the advantage that they tend to adhere well to the metal stents and can withstand significant deformation without cracking. The high elongation and good adhesion provide superior performance to other polymer coatings when the coated stent is expanded. Examples of suitable bioabsorbable elastomers are described in U.S. Patent No. 5,468,253. Preferably the bioabsorbable biocompatible elastomers based on aliphatic polyester, including but not limited to those selected from the group consisting of elastomeric copolymers of epsilon-caprolactone and glycolide (preferably having a mole ratio of epsilon -caprolactone to glycolide of from about 35:65 to about 65:35, more preferably 45:55 to 35:65) elastomeric copolymers of E-caprolactone and lactide, including L-lactide, D-lactide blends thereof or lactic acid copolymers (preferably having a mole ratio of epsilon -caprolactone to lactide of from about 35:65 to about 90:10 and more preferably from about 35:65 to about 65:35 and most preferably from about 45:55 to 30:70 or from about 90:10 to about 80:20) elastomeric copolymers of p-dioxanone (1,4-dioxan-2-one) and lactide including L-lactide, D-lactide and lactic acid (preferably having a mole ratio of p-dioxanone to lactide of from about 30:70 to about 70:30, 45:55 to about 55:45, and preferably from about 40:60 to about 60:40) elastomeric copolymers of epsilon - caprolactone and p-dioxanone (preferably having a mole ratio of epsilon -caprolactone to p-dioxanone of from about 40:60 to about 60:40 and preferably from about 30:70 to about 70:30) elastomeric copolymers of p-dioxanone and trimethylene carbonate (preferably having a mole ratio of p-dioxanone to trimethylene carbonate of from about 40:60 to about 60:40, and preferably from about 30:70 to about 70:30), elastomeric copolymers of trimethylene carbonate and glycolide (preferably having a mole ratio of trimethylene carbonate to glycolide of from about 40:60 to about 60:40 and preferably from about 30:70 to about 70:30), elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. As is well known in the art these aliphatic polyester copolymers have different hydrolysis rates, therefore, the choice of elastomer may in part be based on the requirements for the coatings adsorption. For example epsilon-caprolactone-co-glycolide copolymer (45:55 mole percent, respectively) films lose 90% of their initial strength after 2 weeks in simulated physiological buffer whereas the epsilon -caprolactone-co-lactide copolymers (40:60 mole percent, respectively) loses all of its strength between 12 and 16 weeks in the same buffer. Mixtures of the fast hydrolyzing and slow hydrolyzing polymers can be used to adjust the time of strength retention.

The amount of coating may range from 0.5 to 20 as a percent of the total weight of the stent after coating and preferably will range from 1 to 15 percent. The polymer coatings may be applied in one or more coating steps depending on the amount of polymer to be applied. Different polymers may also be used for different layers in the stent coating. In fact it may be an option to use a dilute first coating solution as primer to promote adhesion of a subsequent coating layers that may contain paclitaxel and melatonin.

Additionally, a top coating can be applied to further delay release of paclitaxel and melatonin, or they could be used as the matrix for the delivery of a different pharmaceutically active material. The amount of top coatings on the stent may vary, but will generally be less than about 2000 micrograms, preferably the amount of top coating will be in the range of micrograms to 1700 micrograms and most preferably in the range of from 300 micrograms to 1000 micrograms. Layering of coating of fast and slow hydrolyzing copolymers can be used to stage release of the drug or to control release of different agents placed in different layers. Polymer blends may also be used to control the release rate of different agents or to provide desirable balance of coating (i.e. elasticity, toughness etc.) and drug delivery characteristics (release profile). Polymers with different solubilities in solvents can be used to build up different polymer layers that may be used to deliver different drugs or control the release profile of a drug. For example since epsilon -caprolactone-co-lactide elastomers are soluble in ethyl acetate and epsilon -caprolactone-co-glycolide elastomers are not soluble in ethyl acetate. A first layer of epsilon -caprolactone-co-glycolide elastomer containing a drug can be over coated with epsilon -caprolactone-co-glycolide elastomer using a coating solution made with ethyl acetate as the solvent. Additionally, different monomer ratios within a copolymer, polymer structure or molecular weights may result in different solubilities. For example, 45/55 epsilon -caprolactone-co-glycolide at room temperature is soluble in acetone whereas a similar molecular weight copolymer of 35/65 epsilon -caprolactone-co-glycolide is substantially insoluble within a 4 weight percent solution. The second coating (or multiple additional coatings) can be used as a top coating to delay the drug delivery of the drug contained in the first layer. Alternatively, the second layer could contain either paclitaxel or melatonin to provide for sequential delivery. Multiple layers of could be provided by alternating layers of first one polymer then the other. As will be readily appreciated by those skilled in the art numerous layering approaches can be used to provide the desired drug delivery.

The coatings can be applied by suitable methodology known to the skilled person, such as, for example, dip coating, spray coating, electrostatic coating, melting a powered form onto the stent. The coating may also be applied during the intervention by the interventional cardiologist on a bare stent. As some polymers (for instance polyorthoesters) need special conservation conditions (argon atmosphere and cold temperature), the drug with the coating may be delivered in a special packing. The MD would apply the coating on the bare stent surface - as it is sligthly sticky - just before introducing the premounted stent inside the patient vessel.

Other examples of polymeric coatings, and coating methods are given in patent documents EP 1 107 707, WO 97/10011, US 6,656,156, EP 0 822 788, US 6,364,903, US 6,231,600, US 5,837,313, WO 96/32907, EP 0 832,655, US 6,653,426, US 6,569,195, EP 0 822 788 B1, WO 00/32238, US 6,258,121, EP 0 832,665, WO 01/37892, US 6,585,764, US 6,153,252.

### Non-polymeric coatings

Another aspect of the invention is a stent coated with a composition of the invention, wherein the presence of a polymer is optional. Such stents suited to polymeric and non-polymeric coatings and compositions are known in the art. These stents may, for example, have a rough surface, microscopic pits or be constructed from a porous material. Examples include, but are not limited to the disclosures of US 6,387,121, US 5,972,027, US 6,273,913 and US 6,099,561.

### Stent grafts

A stent may also be combined with a graft to form a composite medical device, often referred to as a stent graft. Such a composite medical device provides additional support for blood flow through weakened sections of a blood vessel. The graft element made be formed from any suitable material such as, for example, textiles such as nylon, Orlon, Dacron, or woven Teflon, and nontextiles such as expanded polytetrafluroethylene (ePTFE).

Stent grafts of the present invention may be coated with, or otherwise adapted to release the paclitaxel and melatonin of the present invention. Stent grafts may be adapted to release paclitaxel and melatonin by (a) directly affixing to the stent graft a composition according to the invention (*e.g.,* by either spraying the stent graft with a polymer /paclitaxel / melatonin film, or by dipping the implant or device into a polymer/drug solution, or by other covalent or noncovalent means); (b) by coating the stent graft with a substance such as a hydrogel which will in turn absorb a composition according to the invention; (c) by interweaving a composition coated thread into the stent graft (*e.g.,* a polymer which releases the paclitaxel and melatonin formed into a thread into the implant or device; (d) by inserting a sleeve or mesh which is comprised of or coated with a composition according to the present invention; (e) constructing the stent graft itself a composition according to the invention; or (f) otherwise impregnating the stent graft with a composition according to the invention.
The stent graft may be biodegradable, made from, but not limited to, magnesium alloy and starch.

Examples and methods of stent graft coating are provided in patent documents WO 00/40278, and WO 00/56247.

### Stent cavities

It is an aspect of the invention that the stent is provided with a composition of the invention which is present in a cavity formed in the stent. Stent in which cavities are present suitable for the delivery of biologically active material are known in the art, for example, from WO 02/060351 US 6,071,305, US 5,891,108.

### Biodegradable stents

Another aspect of the invention is a biodegradable (bioabsorbable) stent impregnated with a composition according to the present invention. The composition may be coated onto the stent or impregnated into the stent structure, said composition released *in situ* concomitant with the biodegradation of the stent. Suitable materials for the main body of the stent includes, but are not limited to poly(alpha-hydroxy acid) such as poly-L-lactide (PLLA), poly-D-lactide (PDLA), polyglycolide (PGA), polydioxanone, polycaprolactone, polygluconate, polylactic acid-polyethylene oxide copolymers, modified cellulose, collagen or other connective proteins or natural materials, poly(hydroxybutyrate), polyanhydride, polyphosphoester, poly(amino acids), hylauric acid, starch, chitosan, adhesive proteins, co-polymers of these materials as well as composites and combinations thereof and combinations of other biodegradable polymers. Biodegradable glass or bioactive glass is also a suitable biodegradable material for use in the present invention. A composition of the present invention may be incorporated into a biodegradable stent using known methods. Examples of biodegradable stents known in the art, include, but are not limited to the those disclosed in US 2002/0099434, US 6,387,124 B1, US 5,769,883, EP 0 894 505 A2, US 653,312, US 6,423,092, US 6,338,739 and US 6,245,103, EP 1 110 561. Biodegradable stents may also be made from a metal (lanthanide such as, but not limited to magnesium or magnesium alloy), or an association of organic and non-organic material (such as, but not limited to a magnesium based alloy combined with starch).

### Slow release formulation

Another aspect of the invention relates to a composition comprising additives which control release of paclitaxel and melatonin. According to another embodiment of the invention, the composition is a slow release formulation. Accordingly, the stent may be provided with a large or concentrated dose of paclitaxel and melatonin. Once the stent is at the site of treatment, paclitaxel and melatonin are released at a rate determined by the formulation. This avoids the need for frequently replacing stents to maintain a particular dose. Another advantage of a slow release formulation is that the composition diffuses day and night, over several days or weeks.

One embodiment of the present invention is a stent comprising a composition as described herein, wherein said composition further comprises one or more slow release agents. Slow release agents may be natural or synthetic polymers, or reabsorbable systems such as magnesium alloys.

Among the synthetic polymers useful according to a slow release formulation of the invention are poly(glycolic) acid, poly(lactic acid) or in general glycolic- and lactic acid based polymers and copolymers. They also include poly caprolactones and in general, poly hydroxyl alkanoates (PHAs) (poly(hydroxy alcanoic acids) = all polyester). They also include Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate (PMMA), poly acryl amides, poly ester amides, poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, and any other synthetic polymer known to a person skilled in the art. Other synthetic polymers may be made from hydrogels based on activated polyethyleneglycols (PEGs) combined with alkaline hydrolyzed animal or vegetal proteins.

Among the natural derived polymers useful according to a slow release formulation of the invention, are poly aminoacids (natural and non natural), poly β-aminoesters. They also include poly (peptides) such as: albumines, alginates, cellulose / cellulose acetates, chitin / chitosan, collagene, fibrine / fibrinogen, gelatine, lignine. In general, proteine based polymers. Poly (lysine), poly (glutamate), poly (malonates), poly (hyaluronic acids). Poly nucleic acids, poly saccharides, poly (hydroxyalkanoates), poly isoprenoids, starch based polymers, and any other natural derived polymer known to a person skilled in the art.

Other polymers may be made from hydrogels based on activated polyethyleneglycols (PEGs) combined with alkaline hydrolyzed animal or vegetal proteins.

For both synthetic and natural polymers, the invention includes copolymers thereof are included as well, such as linear, branched, hyperbranched, dendrimers, crosslinked, functionalised (surface, functional groups, hydrophilic/hydrophobic).

Preferred slow-release agents include elastomeric, biodegradable poly(ester amide) (PEA) copolymers. These co-PEA polymers have functional carboxyl groups for drug conjugation and are synthesized from non-toxic building blocks. They are prepared using naturally occurring α-amino acids (L-leudine and L-lysine) and other non-toxic building blocks such as 1,6-hexanediol and sebacic acid. Furthermore, the nitroxide radical 4-amino TEMPO can be conjugated to PEA. An important feature of PEA copolymers may be their ability to promote a natural healing response. They posses several desirable characteristics that distinguish this familiy of polymers from other biodegradable polymers:
- Programmable: components of the polymer can be changed to customize biological and physical properties
- Funcionalizable: molecules can be covalently conjugated to the polymer backbone
- Ductile: current formulations for stents have greater than 300% elongation
- Surface degradation: controlled release of matrixed drugs or biologics
- Enzymatic biodegradation: enzymatic attack of the amino acid-like ester and amide bonds
- Blood, cell and tissue compatibility: in vitro, pre-clinical and clinical studies have demonstrated the biocompatibility of PEAs.
Preferred PEA compositions include, but are not restricted to; PEA 8LD60% Tempo; PEA 4LD33% Tempo; PEA Ac Tempo; PEA Ac Bz and PEA GJ-2-2.

The slow release composition may be formulated as liquids or semi-liquids, such as solutions, gels, hydrogels, suspensions, lattices, liposomes.

According to an aspect of the invention, a composition is formulated such that the quantity of melatonin is between less than 1% and 60 % of total slow-release polymer mass. According to an aspect of the invention, a composition is formulated such that the quantity of melatonin is between 1% and 50%, 1% and 40%, 1 % and 30%, 1 % and 20%, 2% and 60%, 5% and 60%,10% and 60%, 20% and 60%, 30% and 60%, or 40% and 60% of total slow-release polymer mass.

According to an aspect of the invention, a composition is formulated such that the quantity of paclitaxel is between less than 1% and 60 % of total slow-release polymer mass. According to an aspect of the invention, a composition is formulated such that the quantity of paclitaxel is between 1% and 50%, 1 % and 40%, 1% and 30%, 1 % and 20%, 2% and 60%, 5% and 60%, 10% and 60%, 20% and 60%, 30% and 60%, or 40% and 60% of total slow-release polymer mass.

### Medical treatments

As mentioned above, SMC proliferation such as stenosis, resteonsis and its prevention are susceptible to treatment by a stent according to the present invention. A stent may be placed on or adjacent to the proliferating SMCs, for example, in a vessel such as an artery. The stent of the present invention may be used to prevent or treat stenosis or restenosis in a subject in need of treatment.

The stent may also be placed *in situ* after the removal of proliferating SMCs. For example, after surgical removal of a stenosis in an artery, a stent may be placed in the area of the artery suture to shrink proliferating cells possibly remaining after surgery.

The paclitaxel and melatonin combination may be combined with a slow release agent so that they can act over a period of days to weeks, so avoiding replacement of the stent. Where a biodregradable stent is used, the stent does not need to be removed after treatment.

The present invention is useful for treating any animal in need including humans, livestock, domestic animals, wild animals, or any animal in need of treatment. Examples of an animal is human, horse, cat, dog, mice, rat, gerbil, bovine species, pig, fowl, camelidae species, goat, sheep, rabbit, hare, bird, elephant, monkey, chimpanzee etc. An animal may be a mammal.

### Paclitaxel

Paclitaxel refers to paclitaxel, analogues and derivatives thereof, including, for example, a natural or synthetic functional analogue of paclitaxel which has paclitaxel biological activity, as well as a fragment of paclitaxel having paclitaxel biological activity. Paclitaxel includes the compound having formula (I). A compound which is a paclitaxel analogue refers to a compound which interferes with cellular mitosis by affecting microtubule formation and/or action, thereby producing antimitotic and anti-cellular proliferation effects.

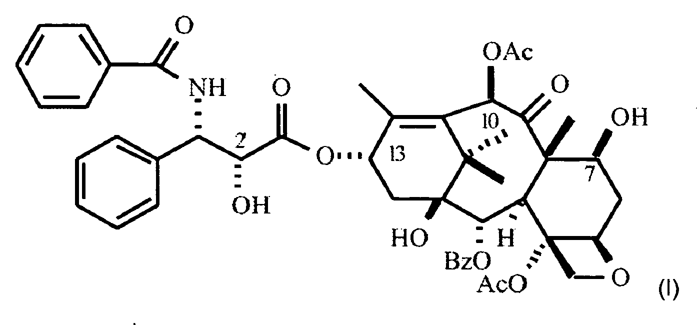

Methods of preparing paclitaxel and its analogues and derivatives are well-known in the art, and are described, for example, in U.S. Pat. Nos. 5,569,729; 5,565,478; 5,530,020; 5,527,924; 5,484,809; 5,475,120; 5,440,057; and 5,296,506. Paclitaxel and its analogues and derivatives are also available commercially. Synthetic paclitaxel, for example, can be obtained from Bristol-Myers Squibb Company, Oncology Division (Princeton, N.J.), under the registered trademark Taxol®.

An analogue of paclitaxel may have a structure according to formula II, whereby paclitaxel is modified at the C10 position. R may be any of Propionyl, Isobutyryl, Valeryl, Hexanoyl, Octanoyl, Decanoyl, Tridecanoyl, Methoxyacetyl, Methylthioacetyl, Methylsulfonylacetyl Acetoxyacetyl, Ethylformyl, Monosuccinyl, Crotonoyl, Acryloyl, Cyclopropanecarbonyl Cyclobutanecarbonyl, Cyclopentanecarbonyl, Cyclohexanecarbonyl, Hydrocinnamoyl, trans-Cinnamoyl, Phenylacetyl, Diphenylacetyl, Benzoyl 2-Chlorobenzoyl, 3-Chlorobenzoyl, 4-Chlorobenzoyl, 3,4-Dichlorobenzoyl, 3,5-Dichlorobenzoyl, 2,4-Dichlorobenzoyl, 3,5-Dibromobenzoyl, 4-Fluorobenzoyl, 3-Trifluoromethylbenzoyl, 4-Trifluoromethylbenzoyl, 3-Nitrobenzoyl, 4-Nitrobenzoyl, 3-Dimethylaminobenzoyl, 3-Methoxybenzoyl, 1-Naphthoyl, 2-Naphthoyl, 2-Quinolinecarbonyl, 3-Quinolinecarbonyl, 4-Quinolinecarbonyl, Indole-3-acetyl, Pyrrole-2-carbonyl, 1-Methyl-2-pyrrolecarbonyl, 2-Furoyl, 5-Bromofuroyl, 5-Nitrofuroyl, 3-Thiophenecarbonyl, 2-Thiophenecarbonyl, 2-Thiopheneacetyl, Picolinoyl, Isonicotinoyl, 5,6-Dichloronicotinoyl, 2-Methylnicotinoyl, 6-Methylnicotinoyl, 5-Bromonicotinoyl, 2-Pyrazinecarbonyl, Isobutyryl, Valeryl, Methoxyacetyl or Cyclohexanecarbonyl. Methods for the preparation of said analogues are described fully in Liu et al, Combinatorial chemistry and High Throughput Screening, 2002, Vol 5, p 39 to 48.

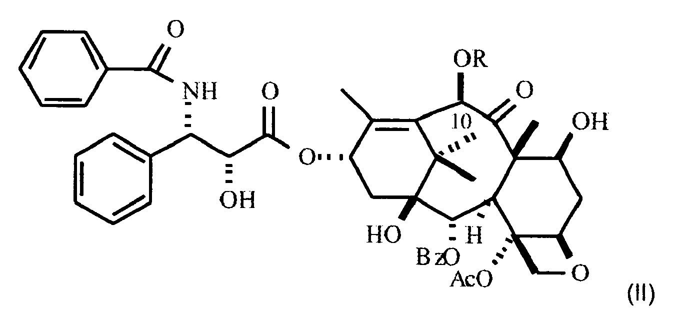

Taxol® and its analogues and derivatives have been used successfully to treat leukemias and tumors. In particular, Taxol® is useful in the treatment of breast, lung, and ovarian cancers. Moreover, paclitaxel may be synthesized in accordance with known organic chemistry procedures (Nerenberg et al., Total synthesis of the immunosuppressive agent (-)-discodermolide. J. Amer. Chem. Soc., 115:12,621-12,622, 1993) that are readily understood by one skilled in the art.

### Melatonin

Melatonin (N-acetyl-5-methoxytryptamine) is a hormone secreted by the pineal gland. Melatonin is often prescribed for the treatment of sleep disturbances and jet-lag. The pharmacological activity of melatonin has been described in numerous publications. One of the early investigations of the pharmacological activity of melatonin was by Barchas and coworkers (Barchas et al. Nature 1967, 214, 919). Melatonin refers to melatonin, analogues and derivatives thereof. An analogue of melatonin is a natural or synthetic functional variant of melatonin which has melatonin biological activity. An analogue of melatonin can be a compound which binds to the melatonin receptor; methods for identifying such melatonin analogues, for example by standard screening techniques, are well known in the art. An analogue may be a compound that binds to the melatonin receptor with an affinity better than 10⁻⁶M in suitable buffer conditions.

Melatonin includes the compound having formula (III).

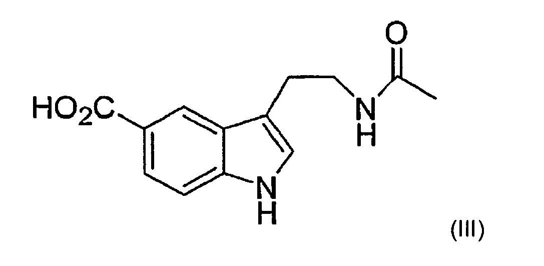

Examples of analogues of melatonin include 2-iodomelatonin, 6-chloromelatonin, 6,7-dichloro-2-methylmelatonin and 8-hydroxymelatonin, all of which contain the 5-methoxy indole ring as an essential moiety (Dubocovich, et al. Proc. Nat'l.Acad. Sci. (USA) 1987, 84, 3916-3918; Dubocovich, M; . J. Pharmacol. Exp.Ther : 1985,234,395; Dubocovich, M. L. Trends Pharmacol. Sci. 1995, 16, 50-56).

### Combinations of analogues

According to one aspect the invention, the paclitaxel comprises one or more analogues of paclitaxel optionally in combination with paclitaxel.

According to another aspect the invention, the melatonin comprises one or more analogues of melatonin optionally in combination with melatonin.

Owing to the properties of proliferating SMCs, the inventors find that a composition comprising combinations of analogues may also be effective at reducing a proliferating cell mass.

### Derivatives

Stereoisomer, tautomers, racemates, prodrugs, metabolites, pharmaceutically acceptable salts, bases, esters, structurally related compounds or solvates of palitaxel or melatonin are within the scope of the invention, unless otherwise stated.

The pharmaceutically acceptable salts of palitaxel or melatonin according to the invention, *i.e.* in the form of water-, oil-soluble, or dispersible products, include the conventional non-toxic salts or the quaternary ammonium salts which are formed, *e.g.,* from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such a sarginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

The term "stereoisomer", as used herein, defines all possible compounds made up of the same atoms bonded by the same sequence of bonds but having different three-dimensional structures which are not interchangeable, which palitaxel or melatonin may possess. Unless otherwise mentioned or indicated, the chemical designation of palitaxel or melatonin herein encompasses the mixture of all possible stereochemically isomeric forms, which said compound may possess. Said mixture may contain all diastereomers and/or enantiomers of the basic molecular structure of said compound. All stereochemically isomeric forms of palitaxel or melatonin either in pure form or in admixture with each other are intended to fall within the scope of the present invention.

Palitaxel or melatonin may also exist in their tautomeric forms. Such forms, although not explicitly indicated in the compounds described herein, are intended to be included within the scope of the present invention.

For therapeutic use, the salts of palitaxel or melatonin according to the invention are those wherein the counter-ion is pharmaceutically or physiologically acceptable.

The term "pro-drug" as used herein means the pharmacologically acceptable derivatives such as esters, amides and phosphates, such that the resulting *in vivo* biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Pro-drugs of the compounds of the invention can be prepared by modifying functional groups present in said component in such a way that the modifications are cleaved, either in routine manipulation or *in vivo,* to the parent component. Typical examples of pro-drugs are described for instance in WO 99/33795, WO 99/33815, WO 99/33793 and WO 99/33792. Pro-drugs are characterized by increased bio-availability and are readily metabolized into the active palitaxel or melatonin *in vivo.*

### Dose

The melatonin and paclitaxel on a stent is preferably present in an amount to inihibit proliferation of smooth muscle cells. It is preferably present in an amount to prevent or treat stenosis or restenosis.

An amount of melatonin and paclitaxel that is effective to prevent or treat stenosis or restenosis is an amount that is effective to ameliorate or minimize the clinical impairment or symptoms of the stenosis or restenosis. For example, the clinical impairment or symptoms of stenosis or restenosis may be ameliorated or minimized by diminishing any pain or discomfort suffered by the subject; by extending the survival of the subject beyond that which would otherwise be expected in the absence of such treatment; by inhibiting or preventing the development or spread of stenosis or restenosis; or by limiting, suspending, terminating, or otherwise controlling the maturation and proliferation of cells in stenosis or restenosis.

The size of stent and concentration of composition thereon will vary depending on the particular factors of each case, including the type of stenosis or restenosis, the stage of stenosis or restenosis, the subject's weight, and the severity of the subject's condition. These amounts can be readily determined by the skilled artisan.

According to one aspect of the invention, a stent is coated with a composition comprising paclitaxel such that the paclitaxel concentration delivered to a subject is greater than or equal to 0.00001, 0.00005, 0.0001, 0.0002, 0.0004, 0.0006, 0.0008, 0.001, 0.002, 0.003, 0.004, 0.005, 0.006, 0.007, 0.008, 0.009, 0.01, 0.05, 0.1, 0.5, 1, 5, 10, 20, 40, 60, 80, or 100 micrograms paclitaxel / mm² of stent, or a concentration in the range between any two of the aforementioned values inclusive.

According to another aspect of the invention, a stent is coated with a composition comprising melatonin such that the melatonin concentration delivered to a subject is greater than or equal to 0.00001, 0.00005, 0.0001, 0.0002, 0.0004, 0.0006, 0.0008, 0.001, 0.002, 0.003, 0.004, 0.005, 0.006, 0.007, 0.008, 0.009, 0.01, 0.05, 0.1, 0.5, 1, 5, 10, 20, 40, 60, 80 or 100 micrograms paclitaxel / mm² of stent, or a concentration in the range between any two of the aforementioned values inclusive.

The concentration of melatonin and paclitaxel per mm² of stent required to arrive at the above doses can be readily calculated by the skilled person.

According to one aspect of the invention, the concentration of melatonin present on a stent may be between 0.001 and 5, 0.02 and 4, or 0.005 and 2 micrograms inclusive melatonin / mm²; preferably it is between 0.005 and 2 micrograms inclusive melatonin /mm² of stent.

According to another aspect of the invention, the concentration of paclitaxel on a stent may be between 0.0001 and 0.5, 0.0005 and 0.003, or 0.001 and 0.2 micrograms inclusive paclitaxel / mm²; preferably it is between 0.001 and 0.2 micrograms inclusive paclitaxel mm².

Preferably, the concentration of paclitaxel on a stent is lower than the concentration of melatonin. The concentration of paclitaxel may be equal to or less than 0.95, 0.9, 0.85, 0.8, 0.75, 0.7, 0.65, 0.6, 0.55, 0.50, 0.45, 0.40, 0.35, 0.30, 0.2, 0.1, 0.05 times the concentration of melatonin, or may be a fraction of the melatonin concentration which fraction is in the range between any two of the aforementioned values inclusive. Preferably it is between 0.1 to 0.3 times the concentration of melatonin. Experiments by the present inventors indicate inhibition occurs with 0.885 micrograms melatonin / mm² stent with a paclitaxel concentration of 0.118 micrograms / mm² stent.

### Kit

A kit according to the invention may comprise at least one stent and separately, at least one composition of the present invention. The kit enables a technician or other person to coat a stent with a composition prior to insertion into a vessel.

The composition, besides comprising melatonin and paclitaxel, may contain additional substances that facilitate the coating of the stent by the end-user. The composition may contain, for example, fast evaporating solvents so as to allow the rapid drying of the stent. It may contain polymeric material to allow the melatonin and paclitaxel to adhere to the stent and facilitate its slow release.

The composition may be applied to the stent of the kit by any means known in the art. For example, by dipping the stent in the composition, by spraying the stent with the composition, by using electrostatic forces. Such methods are known in the art.

It is an aspect of the invention that the composition is provided in a container. For example, a vial, a sachet, a screw-cap bottle, a syringe, a non-resealable vessel, a resealable vessel. Such containers are any that are suitable for containing a composition and optionally facilitating the application of the composition to the stent. Indeed, some polymers to be used for the coating and the controlled release of the active compound, such as polyorthoesthers, are extremely unstable, are very sensitive to humidity and should be conserved in a cold atmosphere and in an argon atmosphere for instance. Some active products as well, such as rotenone, are sensitive to light and heat and should be preserved in dark and cold. In such a case, a container with the composition is kept separately from the bare stent. The interventional cardiologist may open the box containing the coating and apply it on the stent just before the intervention.

A kit may comprise more than one type of stent and more than one container of composition. A kit may provide a range of stent sizes, stent configurations, stents made from different materials. A kit may provide a range of vials containing separately melatonin, paclitaxel, derivatives of melatonin and/or paclitaxel different and different combinations of polymers. A kit may facilitate the sequential application of more than one type of composition. A kit may contain instructions for use.

### EXAMPLES

The invention is illustrated by the following non-limiting examples. They illustrate the effectiveness of the combination of melatonin and paclitaxel described above. The inhibitory properties of the analogues are not mentioned in the examples are known, and the skilled person may readily substitute the exemplified compounds with analogues such as listed above.

### Example 1

A composition comprising between 0.05 and 2 micrograms of melatonin and 0.01 to 0.2 micrograms of paclitaxel per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### Example 2

The aim of the present experiments was to test:
1. Evaluation of post-implantation injury and inflammatory response in a porcine coronary model.
2. Evaluation of the relationship between injury and inflammation, 4 weeks after stent implantation.
3. This study has been set-up to compare coated and non-coated bare stents. The bare metal stainless steel stent is a reference stent and serves as a control group.

### 1. Methods

### 1.1 Stent / balloon coating

Stents were spray-coated in layers with a matrix layer containing a mixture of melatonin /paclitaxel / PEA and a toplayer solely composed of PEA. All handling procedures and spray-coating were performed under cleanroom conditions. Stents were extensively dried under vacuum to remove any solvent residues.

### 1.2 Trial overview

Coronary injury and consequent peri-stent inflammation, peri-strut thrombus formation, and neointimal proliferation were studied at day 28 after implantation of coronary stents in porcine coronary arteries. According to a randomization list, stents were implanted into LAD and CX arteries of domestic pigs:
Group Code:
   Group 1: Stainless Steel (SS)
   Group 5: PEA-Melatonin 150µg (M)
   Group 7: Taxus stent (P)
   Group 8: PEA-Pacl-Mel 20 µg 150 µg 2-layer coating (PM1)
   Group 9: PEA-Pacl-Mel 20 µg 150 µg 3-layer coating (PM2)

### 1.3 Randomization list

The following codes were given to animals provided with a stent in Left Anterior Descending (LAD) artery or a stent in the circumflex (CX) artery in a randomized trial:

**TABLE 1: Animal ID codes, assigned stents (PEA - poly(ester amide); Pacl - paclitaxel; Mel - melatonin; Taxus stent - manufactured by Boston Scientific disposed with paclitaxel), and placement of stent in LAD (Left Anterior Descending) artery or CX (circumflex) artery. Codes used herein: SS - stainless steel stent i.e. provided with no drug, M - melatonin drug eluting stent, PM - melatonin / paclitaxel drug eluting stent, P - paclitaxel drug eluting stent e.g. Taxus stent.**

| **Animal ID** | **LAD Stent** | **CX Stent** |
|---|---|---|
| G66/11 | Stainless Steel (SS stent) | |
| G66/12 | Stainless Steel (SS stent) | |
| G66/13 | Stainless Steel (SS stent) | |
| G66/14 | Stainless Steel (SS stent) | |
| G66/15 | | Stainless Steel (SS stent) |
| G66/16 | | Stainless Steel (SS stent) |
| G66/17 | | Stainless Steel (SS stent) |
| G66/18 | | Stainless Steel (SS stent) |
| G66/27 | PEA-Melatonin 150µg (M stent) | |
| G66/28 | PEA-Melatonin 150µg (M stent) | |
| G66/29 | PEA-Melatonin 150µg (M stent) | |
| G66/30 | PEA-Melatonin 150µg (M stent) | |
| G66/31 | | PEA-Melatonin 150µg (M stent) |
| G66/32 | | PEA-Melatonin 150µg (M stent) |
| G66/33 | | PEA-Melatonin 150µg (M stent) |
| G66/34 | | PEA-Melatonin 150µg (M stent) |
| G66/35 | Taxus stent (P stent) | PEA-Pacl-Mel 20 µg 150 µg 3-layer coating (PM stent) |
| G66/36 | Taxus stent (P stent) | PEA-Pacl-Mel 20 µg 150 µg 3-layer coating (PM stent) |
| G66/37 | Taxus stent (P stent) | PEA-Pacl-Mel 20 µg 150 µg 3-layer coating (PM stent) |
| G66/38 | Taxus stent (P stent) | PEA-Pacl-Mel 20 µg 150 µg 3-layer coating (PM stent) |
| G66/39 | PEA-Pacl-Mel 20 µg 150 µg 2-layer coating (PM stent) | Taxus stent (P stent) |
| G66/40 | PEA-Pacl-Mel 20 µg 150 µg 2-layer coating (PM stent) | Taxus stent (P stent) |
| G66/41 | PEA-Pacl-Mel 20 µg 150 µg 2-layer coating (PM stent) | Taxus stent (P stent) |
| G66/42 | PEA-Pacl-Mel 20 µg 150 µg 2-layer coating (PM stent) | Taxus stent (P stent) |
| G66/43 | PEA-Pacl-Mel 20 µg 150 µg 3-layer coating (PM stent) | PEA-Pacl-Mel 20 µg 150 µg 2-layer coating (PM stent) |
| G66/44 | PEA-Pacl-Mel 20 µg 150 µg 3-layer coating (PM stent) | PEA-Pacl-Mel 20 µg 150 µg 2-layer coating (PM stent) |
| G66/45 | PEA-Pacl-Mel 20 µg 150 µg 3-layer coating (PM stent) | PEA-Pacl-Mel 20 µg 150 µg 2-layer coating (PM stent) |
| G66/46 | PEA-Pacl-Mel 20 µg 150 µg 3-layer coating (PM stent) | PEA-Pacl-Mel 20 µg 150 µg 2-layer coating (PM stent) |

### 1.4 First intervention

All animals of the experiment received equal treatment. First intervention contained the following measurements:
- Coronary angiography
- Implantation of coronary stents in the two main vessels of the left coronary artery (LAD and CX)

The pigs were sedated with 0.2 ml/kg Ketamin (Ursotamin ®, Serumwerk Bernburg, Beerse, Germany) plus 0.1 ml/kg Xylazinhydrochlorid 2% (Xylazin ®, Riemser Arzneimittel GmbH, Germany) plus 0.08 ml/kg Atropinsulfat (Atropinsulfat ®, BBraun Melsungen AG, Germany) before general anaesthesia were induced with intravenous 3-5 ml Propofol (Recofol ®1%, Curamed Pharma GmbH, Germany). The pigs were intubated (Endonorm 6.0 F, Rüsch GmbH, Germany) and ventilation were started using a mixture of 30 vol% of pure oxygen, 70 vol% N₂O and 1-2 vol% of Isofluran (Isofluran Curamed, Curamed Pharma GmbH, Germany). Throughout the procedure, the electrocardiogram, SpO2 and temperature were monitored continuously. An external carotid artery was surgically exposed and an 6F intra-arterial sheath was introduced over a 0.035" guide wire.

Heparin (5000 IU) and 250 mg DL-Lysinmono(acetylsalicylat) (Aspisol ®, Bayer Ag, Germany) were administered intravenously as a bolus. Coronary imaging was done using a Philips PolyArc fluoroscope connected to a digitizer using an Apple Macintosh Power PC. The pig coronary arteries were visualised using an 6F Judkins L3.5 SH catheter, and lopromid (Ultravist 370 ®, Schering AG, Germany) was used as contrast agent. The stents (two in each pig, one coated and one non-coated) were implanted using an oversizing between 10-20%. The I.D. number of every stent was documented for each implantation. Coronary angiograms were performed after administration of nitroglycerin (200 µg). Finally, the carotid arteriotomy were repaired and the dermal layers closed using standard techniques. Anticoagulants were administered 2 days before first intervention until examination. ((250 mg Ticlopidinhydro-chlorid (Tiklyd ®, Sanofi Winthrop, Germany) and 100 mg Acetylsalicylic acid (ASS ®, Ratiopharm, Germany) per day.))

### 1.5 Second intervention

All animals of the experiment received equal treatment. Second intervention contained the following measurements:
- Coronary angiography
- Euthanasia of the animal
- Thoracotomy and explantation of the heart
- Irrigation of the heart with NaCl and pressure fixation with Formalin 3.5%
- Storage of the heart separately in Formalin 3.5 % till histopathologic analysis

The pigs were sedated with 0.2 ml/kg Ketamin (Ursotamin ®, Serumwerk Bernburg, Beerse, Germany) plus 0.1 ml/kg Xylazinhydrochlorid 2% (Xylazin ®, Riemser Arzneimittel GmbH, Germany) plus 0.08 ml/kg Atropinsulfat (Atropinsulfat ®, BBraun Melsungen AG, Germany) before general anaesthesia was induced with intravenous 3-5 ml Propofol (Recofol ®1%, Curamed Pharma GmbH, Germany). The pigs were intubated (Endonorm 6,0 F, Rüsch GmbH, Germany) and ventilation was started using a mixture of 30 vol% of pure oxygen , 70 vol% N2O and 1-2 vol% of Isofluran (Isofluran Curamed, Curamed Pharma GmbH, Germany). Throughout the procedure, the electrocardiogram, SpO2 and temperature were monitored continuously. An external carotid artery was surgically exposed and an 6F intra-arterial sheath was introduced over a 0.035" guide wire.

Coronary imaging was done using a Philips PolyArc fluoroscope connected to a digitizer using an Apple Macintosh Power PC. After follow-up angiography the animals were subsequently sacrificed using an intravenous bolus of 10 ml over saturated potassium chloride (KCI) in deep anaesthesia. Hearts were rapidly excised, the coronary system flushed with 0.9% saline (NaCl) and the arteries fixed by perfusion with 3.5% buffered formalin under physiological pressure and overnight immersion.

The target segments were dissected and samples obtained for histology. The stented coronary arteries were harvested for visual inspection of the stent and histopathologic analysis of the stented artery.

### 1.6 Quantitative coronary analysis

Coronary imaging was performed using a Philips PolyArc fluoroscope connected to a digitizer using an Apple Macintosh Power PC. Quantitative coronary angiography (QCA) was performed with the CAAS II for Research 2.0.1 System (Pie Medical, The Netherlands).

### 1.7 Histology

Hearts were rapidly excised, the coronary system flushed with 0.9% saline and the arteries fixed by perfusion with 4% buffered formalin under physiological pressure and overnight immersion. The hearts were sent to an histology facility at Saarland University. Stented coronary arteries were dissected from the formalin-fixed hearts and immersed in methyl-methacrylate (Merck, Darmstadt, Germany). At least three representative cross sections per stent were cut from the blocks with a coping saw, polished, and glued on acrylic plastic slides. Final specimens were stained by HE, Masson Goldner technique. For endothelialization, von-Willbrand antibody staining was done in selected arteries.

After digitalizing, histomorphometric measurements were taken with the NIH image program (PC version 'Scion Image', Scion Corporation, Maryland, USA). The evaluated parameters were: luminal diameter, external elastic lamina (EEL) diameter, maximal neointimal thickness, EEL area, luminal area, and neointimal area. Injury scores were assigned as previously described by Schwartz et al. (JACC, 1992, Vol 19(2), p. 267 to 274), and the inflammation score for each individual strut was graded as described by Kornowski et al. (JACC, 1998, Vol 31 (1), p. 224 to 230).

### 1.8 Statistical analysis

Angiographic and histomorphometric variables were averaged to obtain a mean value per stent. Continuous variables of quantitative coronary angiography were compared by an analysis of variance model with pig as random factor and treatment, vessel and treatment-vessel interaction as fixed factors using the software SPSS 12.0 for Windows (SPSS Inc., Chicago, IL, USA). Data will be presented as the mean value ± SD. A p value of less than or equal to 0.05 is considered as statistically significant.

### 2. Results

### 2.1 Imaging angiography and histology

A summary of imaging and histology results given in Table 2 below, which refers to images presented in the Figures.

**TABLE 2. Imaging and histology results for each animal.**

| **Animal** ID | **Angiography images** | **LAD histology images** | **Stent implant(s)** |
|---|---|---|---|
| G66/11 | Figure 1 | Figure 2 | SS (LAD) |
| G66/12 | Figure 3 | Figure 4 | SS (LAD) |
| G66/13 | Figure 5 | Figure 6 | SS (LAD) |
| G66/14 | Figure 7 | Figure 8 | SS (LAD) |
| G66/15 | Figure 9 | Figure 10 | SS (CX) |
| G66/16 | Figure 11 | Figure 12 | SS (CX) |
| G66/17 | Figure 13 | Figure 14 | SS (CX) |
| G66/18 | Figure 15 | Figure 16 | SS (CX) |
| G66/27 | Figure 47 | Figure 48 | M (LAD) |
| G66/28 | Figure 49 | Figure 50 | M (LAD) |
| G66/29 | Figure 51 | Figure 52 | M (LAD) |
| G66/30 | Figure 53 | Figure 54 | M (LAD) |
| G66/31 | Figure 55 | Figure 56 | M (CX) |
| G66/32 | Figure 57 | Figure 58 | M (CX) |
| G66/33 | Figure 59 | Figure 60 | M (CX) |
| G66/34 | Figure 61 | Figure 62 | M (CX) |
| G66/35 | Figure 17 | Figure 18,19 | P (LAD) / PM (CX) |
| G66/36 | Figure 20 | Figure 21, 22 | P (LAD) / PM (CX) |
| G66/37 | Figure 23 | Figure 24, 25 | P (LAD) / PM (CX) |
| G66/38 | Animal died 11 days after procedure | Animal died 11 days after procedure | P (LAD) / PM (CX) |
| G66/39 | Figure 26 | Figure 27, 28 | PM (LAD) / P (CX) |
| G66/40 | Figure 29 | Figure 30, 31 | PM (LAD) / P (CX) |
| G66/41 | Figure 32 | Figure 33, 34 | PM (LAD) / P (CX) |
| G66/42 | Figure 35, | Figure 36, 37 | PM (LAD) / P (CX) |
| G66/43 | Figure 38 | Figure 39, 40 | PM (LAD) / PM (CX) |
| G66/44 | Animal died 13 days after procedure | Animal died 13 days after procedure | PM (LAD) / PM (CX) |
| G66/45 | Figure 41 | Figure 42, 43 | PM (LAD) / PM (CX) |
| G66/46 | Figure 44 | Figure 45, 46 | PM (LAD) / PM (CX) |

### 2.2 Quantitative Coronary Angiography

The drug eluting stents PM1 and PM2 showed a reduced angiographic late lumen loss after 4 weeks as indicated in Table 3. The Taxus ® stent was associated with an increased late loss in this animal model.

**TABLE 3: Summary of quantitative coronary angiography, 4 week follow-up. Comparison of the following groups: group 1 Stainless Steel (SS), group 7 Taxus stent (P), group 5 PEA-Melatonin 150µg (M), group 8 PEA-Pacl-Mel 20 µg 150 µg 2-layer coating (PM1), and group 9 PEA-Pacl-Mel 20 µg 150 µg 3-layer coating (PM2).**

| | **SS** | **P** | **M** | **PM1** | **PM2** | ***p*** |
|---|---|---|---|---|---|---|
| **n** | 8 | 7 | 8 | 7 | 6 | |
| **reference diameter** | 2.00 ± 0.62 | 2.01 ± 0.47 | 2.10 ± 0.34 | 1.94 ± 0.33 | 2.11 ± 0.43 | 0.935 |
| **stent diameter** | 2.32 ± 0.38 | 2.64 ± 0.23 | 2.64 ± 0.19 | 2.35 ± 0.17 | 2.55 ± 0.23 | 0.103 |
| **Overstretch** | 1.22 ± 0.30 | 1.36 ± 0.26 | 1.29 ± 0.21 | 1.24 ± 0.20 | 1.24 ± 0.26 | 0.724 |

| **control angiography** | | | | | | |
|---|---|---|---|---|---|---|
| **RFD control** | 1.91 ± 0.56 | 2.15 ± 0.33 | 2.10 ± 0.38 | 2.03 ± 0.35 | 2.28 ± 0.49 | 0.470 |
| **MLD control** | 1.25 ± 0.74 | 1.18 ± 0.81 | 1.07 ± 0.46 | 1.68 ± 0.34 | 1.87 ± 0.30 | 0.137 |
| **late** lumen loss | 1.07 ± 0.69 | 1.46 ± 0.65 | 1.58 ± 0.40 | 0.67 ± 0.36 | 0.68 ± 0.27 | 0.038 |

### 2.3 Histomorphometry

The drug eluting stents PM1 and PM2 showed a trend towards a reduced neointimal hyperplasia after 4 weeks as indicated in Table 4 below. The Taxus ® stent was associated with an increased neointimal formation in this animal model.

**TABLE 4: Summary of histomorphometry, 4 weeks follow-up. Comparison of the following groups: group 1 Stainless Steel (SS), group 7 Taxus stent (P), group 5 PEA-Melatonin 150µg (M), group 8 PEA-Pacl-Mel 20 µg 150 µg 2-layer coating (PM1), and group 9 PEA-Pacl-Mel 20 µg 150 µg 3-layer coating (PM2).**

| | **SS** | **P** | **M** | **PM1** | **PM2** | **P** |
|---|---|---|---|---|---|---|
| N | 8 | 7 | 8 | 7 | 6 | |
| **vessel diameter [mm]** | 2.68 ± 0.34 | 3.19 ± 0.29 | 3.08 ± 0.23 | 3.07 ± 0.16 | 3.10 ± 0.23 | 0.006 |
| **lumen diameter [mm]** | 1.63 ± 0.40 | 1.79 ± 0.94 | 1.75 ± 0.40 | 2.33 ± 0.22 | 2.53 ± 0.26 | 0.014 |
| **max. neoint. thickn. [mm]** | 0.64 ± 0.14 | 0.75 ± 0.63 | 0.84 ± 0.30 | 0.30 ± 0.23 | 0.13 ± 0.06 | 0.009 |
| **vessel area [mm²]** | 5.74 ± 1.46 | 7.98 ± 1.37 | 7.37 ± 1.10 | 7.51 ± 1.02 | 7.40 ± 1.12 | 0.012 |
| **luminal area [mm²]** | 2.39 ± 1.20 | 3.21 ± 2.48 | 2.54 ± 1.14 | 4.30 ± 0.84 | 4.98 ± 0.99 | 0.021 |
| **neointimal area [mm²]** | 3.35 ± 0.93 | 4.77 ± 2.46 | 4.83 ± 1.19 | 3.20 ± 1.28 | 2.42 ± 0.32 | 0.057 |
| **injury score [-]** | 1.18 ± 0.32 | 1.74 ± 0.84 | 1.69 ± 0.70 | 0.98 ± 0.67 | 0.48 ± 0.50 | 0.012 |
| **inflammation score [-]** | 1.35 ± 0.50 | 1.41 ± 1.19 | 2.05 ± 0.83 | 0.85 ± 0.88 | 0.56 ± 0.50 | 0.201 |

### 3. Conclusion

Stents spray-coated in layers with a the matrix layer containing a mixture of melatonin and paclitaxel lead to a reduction of neointimal formation in the porcine coronary model.

### Example 3

The present experiments aimed to demonstrate that melatonin shows significant inhibitory effects on neointimal formation *in vivo* following percutaneous coronary intervention (PCI) in a pig model. In addition, it aimed to elucidate:
- the action of Melatonin on human vascular cells (coronary artery endothelial and smooth muscle cells) and
- the underlying molecular and cellular mechanisms.

### 1. Materials and methods

### 1.1 Cell culture

Human coronary artery endothelial cells (HCAEC) were purchased from Cambrex and human coronary artery smooth muscle cells (HCASMC) were purchased from Promocell. Cells were used for experiments between passage 4 and passage 12. Both cell types were cultured in their respective complete culture medium with the following composition:
- HCASMC: Smooth Muscle Cell Growth Medium 2: Epidermal Growth Factor (EGF), basic Fibroblast Factor (FGF), Insulin, Fetal Calf Serum (FCS) 5 %, Amphotericin B, Gentamicin.
- HCAEC: Endothelial Growth Media-2 MV: hEGF, Hydrocortisone, GA-1000, FBS 5%, VEGF, hFGF-B, R3-IGF-1, Ascorbic Acid

Medium was changed every other day. 24 hours prior to growth factor stimulation, the medium was replaced by fresh culture medium, however, without growth factors to increase the cellular sensitivity to the subsequent growth factor stimulation. Subconfluent HCAEC were stimulated with 10 ng/ml of VEGF-A, whereas subconfluent HCASMC were stimulated with 10 ng/ml of PDGF-BB. Likewise, both cell types were stimulated with 25% of human serum (final concentration).

To investigate, whether melatonin has similar effects on other cell types, HUVEC (Human Umbilical Vein Endothelial Cells, Promocell) were tested. HUVEC were cultured in the corresponding complete medium (Endothelial Cell Growth Medium: ECGS/H 0.4 %, Fetal Calf Serum 2%, Epidermal Growth Factor 0.1 ng/ml, Hydrocortison 1 µg/ml, basic Fibroblast Factor 1 ng/ml, Amphotericin B 50 ng/ml, Gentamicin 50 µg/ml). 24 hours prior to stimulation, the medium was replaced by a medium containing 2% serum and antibiotics, but devoid of the other complements. Then, HUVEC were stimulated with 10 ng/ml of VEGF-A for 24 hours.

Melatonin (Acros organics) was kindly provided by BlueMedical Devices BV. A stock solution was prepared in DMSO, aliquoted and stored at -20°C. For each experiment a fresh aliquot was opened and diluted to the desired final concentration in cell culture medium.

### 1.2. Proliferation assay

All proliferation and cytotoxicity experiments were performed on cells cultured on 96 well plates (Costar). The CellTiter 96 One solution cell proliferation assay from Promega was used to assess cell proliferation in a 96-well plate. This assay makes use of the MTS tetrazolium compound as a cellular substrate. MTS is bioreduced by living cells into a coloured formazan product that is soluble in tissue culture medium and absorbs light at 490 nm. The quantity of formazan produced is directly proportional to the number of living cells in culture. After 24h stimulation, 20 µl of MTS reagent was added to the cells cultured in 100 ul of medium per well. After 2 to 4 hours of incubation at 37°C, 5% CO₂, the absorbance at 490 nm was recorded using a 96-well plate reader (Tecan).

### 1.3. Cytotoxicity assay

Cytotoxicity experiments were performed with cells cultured on 96-well plates. The CytoTox-ONE^{™} Assay from Promega was used to assess the membrane integrity of cells. This test is based on a fluorometric method to estimate the number of non viable cells present in multiwell plates by measuring the release of lactate dehydrogenase (LDH) from cells with damaged membranes. Subconfluent cells were incubated for 48 hours with Melatonin at concentrations between 0.2 and 6 mM. During the 48h Melatonin incubation, the medium was not changed to avoid removal of any detached cells. As a positive control three wells of untreated cells were incubated with lysis buffer (delivered with the assay) before measurement. At the end of the Melatonin incubation period, a volume of CytoTox-ONE reagent equal to the volume of cell culture medium present in each well was added. After 10 min incubation at 22°C (room temperature), 50 µl of stop solution was added per 100 µl of CytoTox-ONE reagent added. The plate was shaked for 10 seconds and fluorescence was recorded with an excitation wavelength of 560 nm and an emission wavelength of 590 nm (Spectramax M2 fluorescence plate reader, Molecular devices).

### 1.4. [³H]-thymidine incorporation

HUVEC were incubated for 48 hours with Melatonin and stimulated for 24 hours with VEGF-A at 10 ng/ml. After 24 hours of stimulation, the cells were incubated with radioactive [³H]-thymidine. Two hour later, the medium was removed and cell-bound DNA was precipitated using 5% TCA (Trichloroacetic acid). Then, [³H]-thymidine incorporation was measured using a beta-counter.

### 1.5. Chemotaxis

Chemotaxis experiments were performed using a modified Boyden chamber. The apparatus consists of two multi-well chambers separated by a filter containing pores of uniform size. A solution containing a chemokine or chemotactic factor is placed in the lower/bottom chamber and a cell suspension is placed in the upper chamber. Cells can migrate through the pores across the filter and towards the chemoattractant in the lower chamber. Cells that migrated across the filter and remain attached to the lower side of the membrane are counted. Data are expressed in terms of migration index: the number of cells that migrated in response to agonist stimulation compared to the number of cells that migrated randomly, *i.e.* to medium only.

Chemotaxis experiments so far were only performed with HCAEC. For these cells a 25 x 80 mm polycarbonate filter, polyvinyl propylene (PVP) free was used (Whatman) with a pore size of 8 µM. Before migration the filter was coated with collagen. Endothelial cells were incubated with Melatonin at 2 mM prior to and during migration. After 24 hours preincubation with Melatonin, untreated and Melatonin-treated cells were detached with trypsin and a cell suspension (0.5 million cells/ml) was loaded in the upper part of the chamber. Cells were stimulated with VEGF-A at 10 ng/ml. Cells were allowed to migrate for 4 hours at 37°C/5% CO₂. At the end of migration, cells were fixed on the filter, stained with Giemsa and counted using a regular optic microscope.

### 2. Results

### 2.1. Melatonin shows antiproliferative effects on both human coronary artery endothelial cells (HCAEC) and human coronary artery smooth muscle cells (HCASMC)

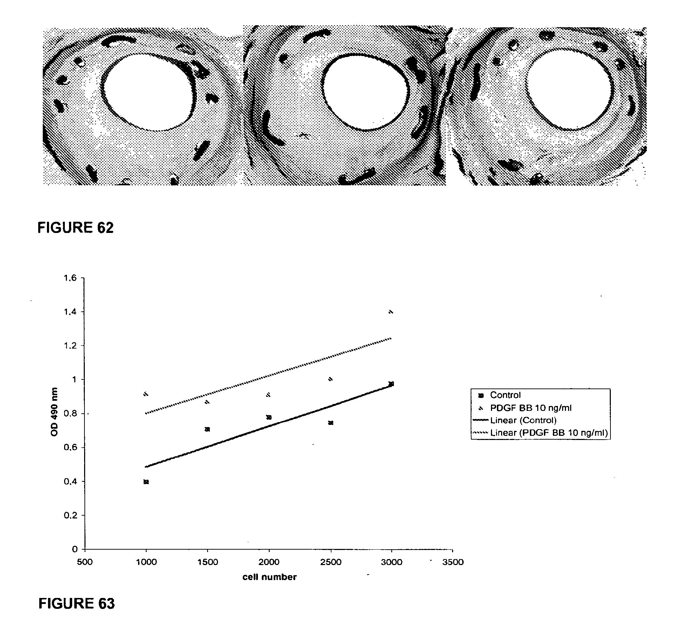

In the first experiment, the proliferation kit was calibrated for smooth muscle cells. HCASMC were seeded at different concentrations. After 4 days, the cells were stimulated with 10 ng/ml of PDGF BB for 48h. The results are presented in Fig. 63. Based on the results of this experiment, the decision was taken to perform all following experiments with a cell concentration for seeding of 2500 cells per well.

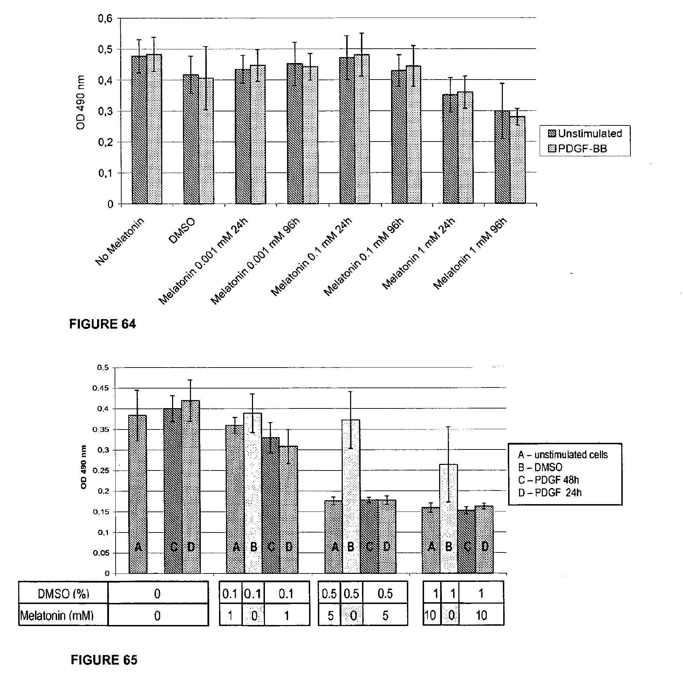

The effect of Melatonin was then tested on HCASMC at concentrations ranging from 1 µM to 10 mM, and was applied either 24h or 96 hours prior to growth factor stimulation. Melatonin was also present during stimulation with growth factors. Cells were stimulated for 24h with 10 ng/ml of PDGF BB. A concentration of Melatonin up to 0.1 mM showed no significant effect on the proliferation of HCASMC (Fig. 64). A 24 hours pre-treatment of the cells with 1 mM of Melatonin induced a significant decrease in the number of smooth muscle cells. This effect was even more pronounced, when the incubation time of Melatonin was increased up to 96 hours prior stimulation. The Melatonin-induced inhibition could not be counteracted by stimulation with PDGF-BB. This is the first description of a time-dependent inhibition of Melatonin on HCASMC.

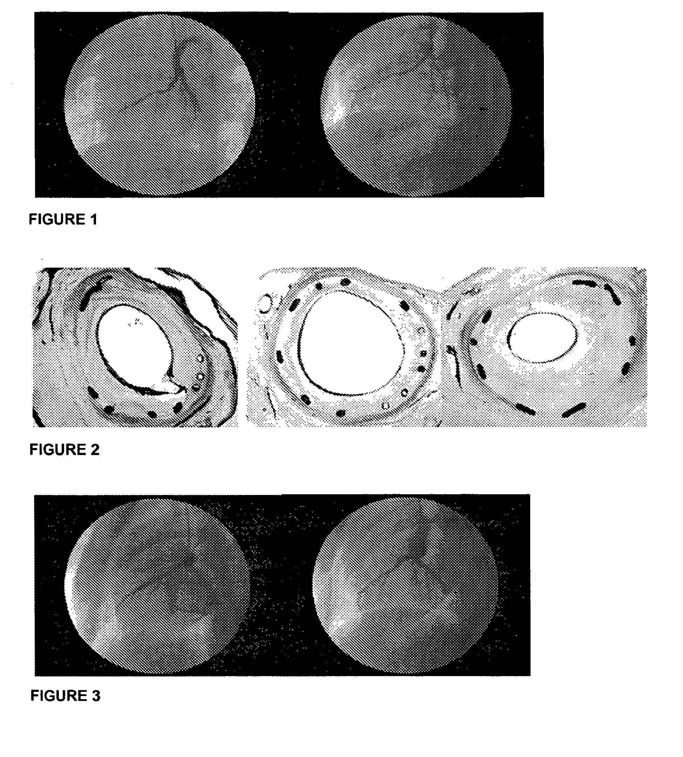

Stimulation of the cells with 25% of human serum led to similar results (data not shown). The maximal inhibitory effect of Melatonin was observed at 5 mM (Fig. 3). At higher concentrations (up to 10 mM), no significant further increase in the inhibitory effect could be obtained.

Melatonin was then tested on human coronary artery endothelial cells (HCAEC). The cells were first seeded at a concentration of 3000 cells per well, but it appeared that this concentration was too high to perform an experiment over a period of 4 days. For all following experiments, the cells were therefore seeded at a concentration of 1000 cells per well.

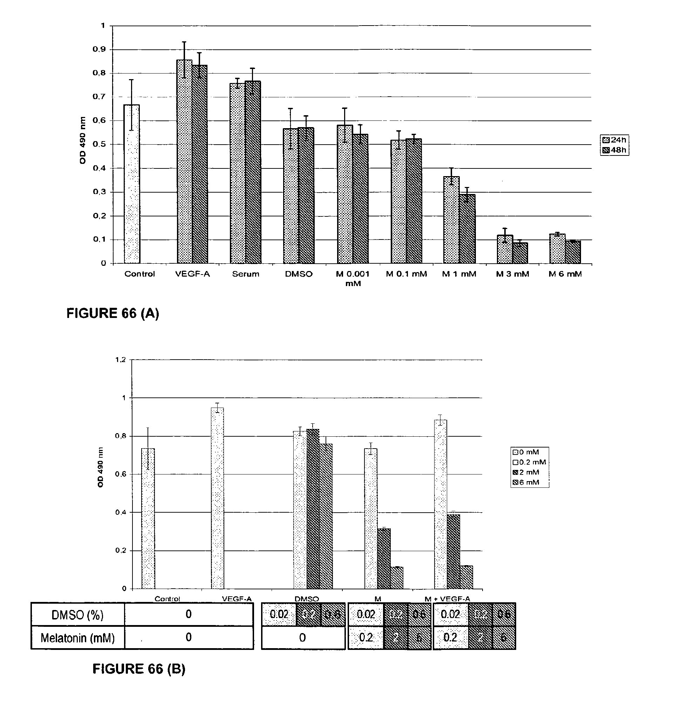

Melatonin was tested at concentrations between 1 µM and 6 mM on subconfluent cells (Fig. 66A). The results obtained with HCAEC were comparable to those obtained with HCASMC. Up to a concentration of 0.1 mM of Melatonin, the HCAEC were not sensitive to Melatonin. At Melatonin concentrations between 1 mM and 6 mM, a dramatic decrease in cell number could be observed with a maximal effect seen at 3 mM and 6 mM. In general, HCAEC appeared to be more sensitive to Melatonin than HCASMC. A stimulation with VEGF A (10 ng/ml) did not overcome the inhibitory effect induced by Melatonin (Fig. 66 B).

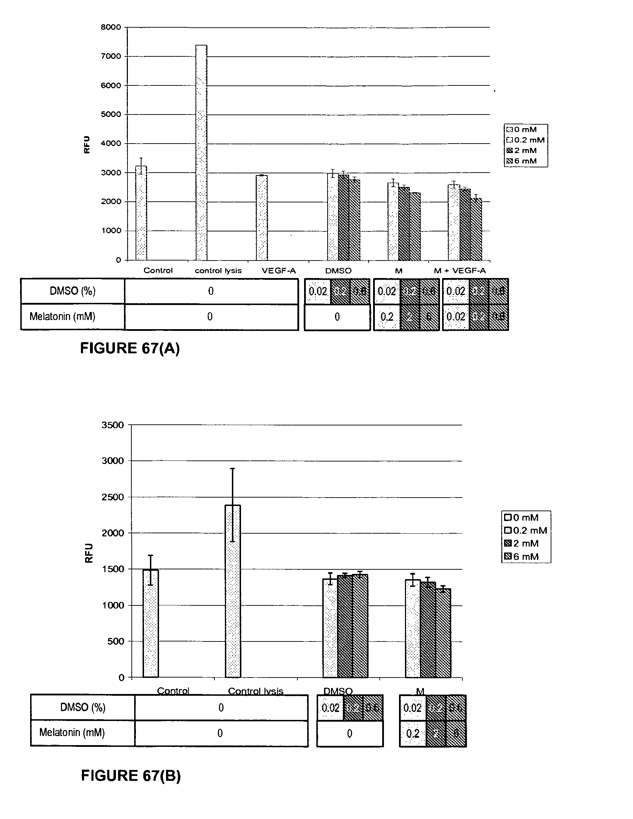

Taken together, these results show that Melatonin induces a decrease in cell number. Two reasons for this effect are possible: 1) Either Melatonin acts as an anti proliferative compound or 2) Melatonin is cytotoxic for the cells. To answer this question, HCAEC and HCASMC were treated with Melatonin and their membrane integrity was subsequently tested using the CytoTox-one assay from Promega (Fig. 67).

### 2.2. The inhibitory effect of Melatonin on HCAEC and HCASMC cannot be explained by a cytotoxic effect

To investigate a possible cytotoxic effect of Melatonin on both HCAEC and HCASMC, the two cell types were treated during 48 hours with increasing Melatonin concentration. The amount of LDH released by dead cells was then measured. The results are presented in Fig. 67.
Based on these results, it can be concluded that Melatonin does not induce any detectable cytotoxic effect on HCAEC (Fig. 67A) nor on HCASMC (Fig. 67B) in this *in vitro* setting.

### 2.3. Melatonin inhibits DNA synthesis of HUVEC

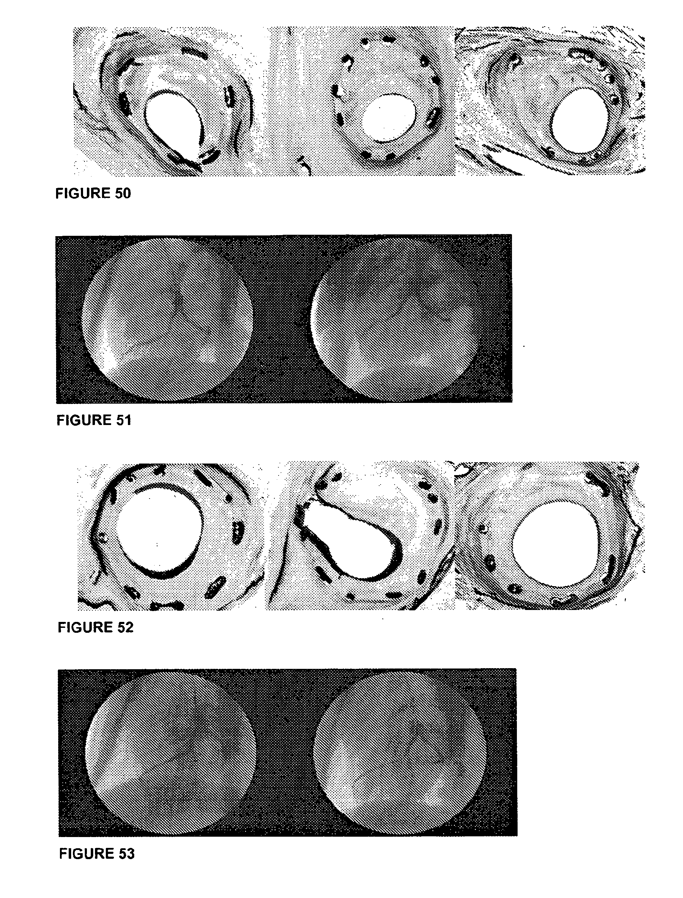

The goal of the following experiment was to investigate, whether Melatonin was able to induce similar effects on HUVEC as it did on HCAEC and HCASMC. For this purpose [3H]-thymidine incorporation experiments were performed on this cell type to assess DNA synthesis and proliferation. The cells were incubated for 48 hours with increasing concentrations of Melatonin and stimulated for 24 hours with 10 ng/ml of VEGF A prior to incorporation of [3H]-thymidine. The results are presented in Fig. 52.

In view of these results, it can be concluded that HUVEC were more sensitive to Melatonin (and to DMSO) compared to HCASMC/HCAEC. Already a concentration of 0.2 mM showed an inhibitory effect on HUVEC, which was not the case for the other two cell types. It cannot be excluded that the number of cells was decreased by Melatonin treatment, but VEGF-A induced [3H]-thymidine incorporation was completely inhibited. The cells were somewhat sensitive to DMSO (negative control), but DMSO-treated cells were still responsive to VEGF-A and incorporated [3H]-thymidine upon VEGF-A-stimulation.

### 2.4. Melatonin does not affect the migratory potential of HCAEC

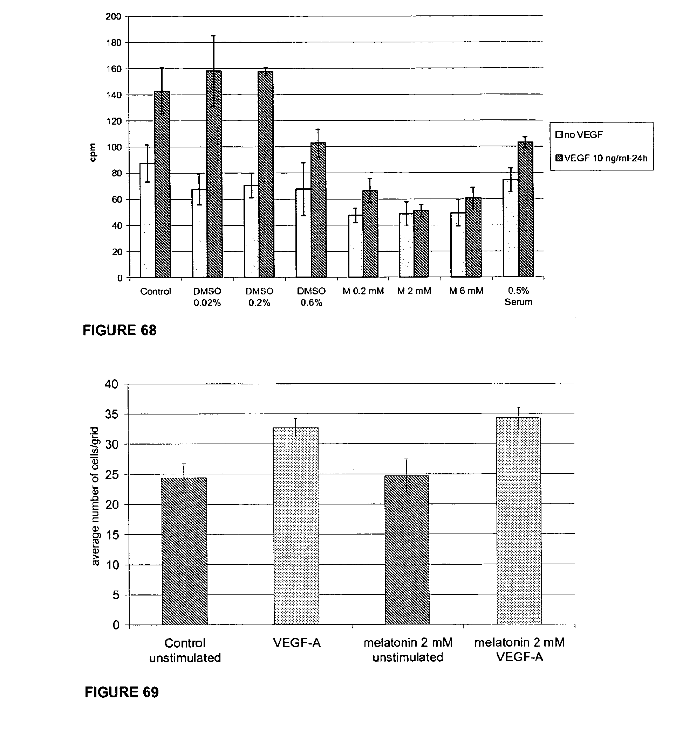

HCAEC were treated with 2 mM of Melatonin for 48 hours and their chemotactic response towards VEGF-A (10 ng/ml) was assessed (migration for 4 hours). The results are presented in Fig. 69.

Melatonin at a concentration of 2 mM, which led to the inhibition of HCAEC proliferation (Fig. 66B), did not have any significant effect on the chemotactic response of these cells. Thus far, this experiment was performed only once and the results need to be confirmed.

### 4. Conclusions

The aim of the experiments was to identify the direct effects of melatonin on human vascular cells and to elucidate the underlying molecular and cellular mechanisms. The results obtained in the first part of the experiments clearly show that melatonin strongly inhibits the proliferation of human coronary artery endothelial cells (HCAEC) and human coronary artery smooth muscle cells (HCASMC) at concentrations higher than 0.2 mM. Melatonin shows no cytotoxic effects so far and does not impair the migratory capacity of human coronary artery endothelial cells. Melatonin has comparable effects on human umbilical vein endothelial cells (HUVEC), which however can be observed at lower concentrations.

The results presented in this study clearly show that Melatonin strongly inhibits the proliferation of HCAEC, HCASMC and HUVEC. Melatonin shows no cytotoxic effects and does not impair the migratory response of endothelial cells towards the endothelial growth factor VEGF-A. These support the the finding that Melatonin is a good drug candidate for the prevention of restenosis following PCI, to be applied in a local drug-delivery setting (drug eluting stent).

## Claims

1. A medical stent provided with a composition comprising melatonin and paclitaxel.

2. Medical stent according to claim 1, wherein said stent is provided with one or more cavities configured to contain and release said composition.

3. Medical stent according to claim 1 or 2, wherein said stent is at least partly made from a material which is biodegradable *in situ.*

4. Medical stent according to any of claims 1 to 3, wherein said stent comprises a magnesium based alloy.

5. Medical stent according to claims 1 or 3, wherein said stent is at least partly made from a material which is non-biodegradable *in situ.*

6. Medical stent according to any of claims 1 to 5, wherein said stent is at least partly provided with said composition.

7. Medical stent according to any of claims 1 to 6, wherein said composition further comprises one or more slow release agents to tune the slow release of the paclitaxel and melatonin.

8. Medical stent according to claim 7, wherein said slow release agent is any of magnesium alloys, poly(glycolic) acid, poly(lactic acid) or in general glycolic- and lactic acid based polymers, copolymers, poly caprolactones and in general, poly hydroxyl alkanoate,s poly(hydroxy alcanoic acids), Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate, poly acryl amides, poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, natural and non natural poly aminoacids , poly β-aminoesters, albumines, alginates, cellulose / cellulose acetates, chitin / chitosan, collagene, fibrine / fibrinogen, gelatine, lignine, proteine based polymers, Poly (lysine), poly (glutamate), poly (malonates), poly (hyaluronic acids), Poly nucleic acids, poly saccharides, poly (hydroxyalkanoates), poly isoprenoids, starch based polymers, copolymers thereof, linear, branched, hyperbranched, dendrimers, crosslinked, functionalised derivatives thereof, or hydrogels based on activated polyethyleneglycols combined with alkaline hydrolyzed animal or vegetal proteins.

9. Medical stent according to claim 7, wherein said slow release agent is a biodegradable poly (ester amide) copolymer.

10. Medical stent according to any of claims 1 to 9 wherein said melatonin is a mixture of at least one melatonin analogue optionally together with melatonin.

11. Medical stent according to any of claims 1 to 10 wherein said paclitaxel is a mixture of at least one paclitaxel analogue optionally together with paclitaxel.

12. Medical stent according to claim 10 or 11 wherein a melatonin analogue is any of 2-iodomelatonin, 6-chloromelatonin, 6,7-dichloro-2-methylmelatonin and 8-hydroxymelatonin.

13. Medical stent according to claim 11 or 12 wherein a paclitaxel analogue is a compound having formula (II), 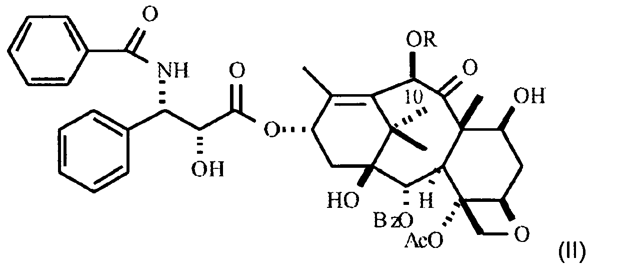 wherein R is any of Propionyl, Isobutyryl, Valeryl, Hexanoyl, Octanoyl, Decanoyl, Tridecanoyl, Methoxyacetyl, Methylthioacetyl, Methylsulfonylacetyl Acetoxyacetyl, Ethylformyl, Monosuccinyl, Crotonoyl, Acryloyl, Cyclopropanecarbonyl Cyclobutanecarbonyl, Cyclopentanecarbonyl, Cyclohexanecarbonyl, Hydrocinnamoyl, trans-Cinnamoyl, Phenylacetyl, Diphenylacetyl, Benzoyl 2-Chlorobenzoyl, 3-Chlorobenzoyl, 4-Chlorobenzoyl, 3,4-Dichlorobenzoyl, 3,5-Dichlorobenzoyl, 2,4-Dichlorobenzoyl, 3,5-Dibromobenzoyl, 4-Fluorobenzoyl, 3-Trifluoromethylbenzoyl, 4-Trifluoromethylbenzoyl, 3-Nitrobenzoyl, 4-Nitrobenzoyl, 3-Dimethylaminobenzoyl, 3-Methoxybenzoyl, 1-Naphthoyl, 2-Naphthoyl, 2-Quinolinecarbonyl, 3-Quinolinecarbonyl, 4-Quinolinecarbonyl, Indole-3-acetyl, Pyrrole-2-carbonyl, 1-Methyl-2-pyrrolecarbonyl, 2-Furoyl, 5-Bromofuroyl, 5-Nitrofuroyl, 3-Thiophenecarbonyl, 2-Thiophenecarbonyl, 2-Thiopheneacetyl, Picolinoyl, Isonicotinoyl, 5,6-Dichloronicotinoyl, 2-Methylnicotinoyl, 6-Methylnicotinoyl, 5-Bromonicotinoyl, 2-Pyrazinecarbonyl, Isobutyryl, Valeryl, Methoxyacetyl or Cyclohexanecarbonyl.

14. Medical stent according to any of claims 1 to 13 wherein the concentration of melatonin present on the stent is between 0.005 and 2 micrograms inclusive melatonin /mm²_{.}

15. Medical stent according to any of claims 1 to 14 wherein the concentration of paclitaxel on the stent is between 0.001 and 0.2 micrograms inclusive paclitaxel / mm².

16. Medical stent according to any of claims 1 to 15 suitable for use in inhibiting SMC proliferation.

17. Medical stent, according to claim 16, wherein said SMC proliferation is restenosis or stenosis.

18. Medical stent according to any of claims 1 to 17 wherein the stent is placed in an artery or vein.

19. Medical stent according to any of claims 1 to 18, wherein the composition comprises melatonin and paclitaxel for separate or simultaneous administration.

20. Medical stent according to claim 19, wherein paclitaxel and melatonin are provided as separate inner and outer layers, one disposed over the other.

21. Medical stent according to claim 20, wherein the inner layer comprises melatonin and the outer layer comprises paclitaxel.

22. Medical stent according to claim 20, wherein the inner layer comprises paclitaxel and the outer layer comprises melatonin.

23. Medical stent according to claim 22, wherein the inner paclitaxel layer and outer melatonin layer are devoid of slow release agents, and said stent is provided with an outermost layer comprising slow release agent.

24. Use of a composition comprising melatonin and paclitaxel, for the preparation of a medicament for providing a medical stent for treating smooth muscle cell, SMC, proliferation.

25. Use according to claim 24, wherein said stent is as defined in any of claims 2 to 6.

26. Use according to claim 24 or 25, wherein said composition further comprises one or more slow release agents to tune slow release of the paclitaxel and melatonin.

27. Use according to claim 26, wherein said slow release agent is any of magnesium alloys, poly(glycolic) acid, poly(lactic acid) or in general glycolic- and lactic acid based polymers, copolymers, poly caprolactones and in general, poly hydroxyl alkanoate,s poly(hydroxy alcanoic acids), Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate, poly acryl amides, poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, natural and non natural poly aminoacids , poly β-aminoesters, albumines, alginates, cellulose /cellulose acetates, chitin / chitosan, collagene, fibrine / fibrinogen, gelatine, lignine, proteine based polymers, Poly (lysine), poly (glutamate), poly (malonates), poly (hyaluronic acids), Poly nucleic acids, poly saccharides, poly (hydroxyalkanoates), poly isoprenoids, starch based polymers, copolymers thereof, linear, branched, hyperbranched, dendrimers, crosslinked, functionalised derivatives thereof, or hydrogels based on activated polyethyleneglycols combined with alkaline hydrolyzed animal or vegetal proteins.

28. Use according to claim 26, wherein said slow release agent is a biodegradable poly (ester amide) copolymer.

29. Use according to any of claims 24 to 28 wherein said melatonin is a mixture of at least one melatonin analogue optionally together with melatonin.

30. Use according to any of claims 24 to 29 wherein said paclitaxel is a mixture of at least one paclitaxel analogue optionally together with paclitaxel.

31. Use according to any of claim 29 or 30 wherein a melatonin analogue is any of 2-iodomelatonin, 6-chloromelatonin, 6,7-dichloro-2-methylmelatonin and 8-hydroxymelatonin.

32. Use according to claim 30 or 31 wherein a paclitaxel analogue is a compound having formula (II), 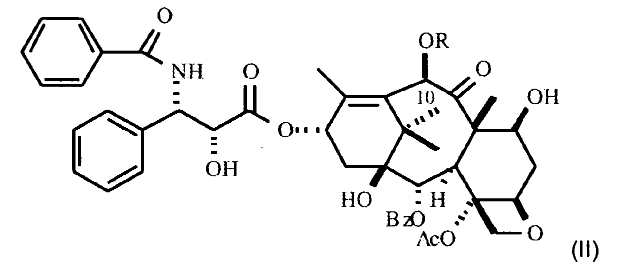 wherein R is any of Propionyl, Isobutyryl, Valeryl, Hexanoyl, Octanoyl, Decanoyl, Tridecanoyl, Methoxyacetyl, Methylthioacetyl, Methylsulfonylacetyl Acetoxyacetyl, Ethylformyl, Monosuccinyl, Crotonoyl, Acryloyl, Cyclopropanecarbonyl Cyclobutanecarbonyl, Cyclopentanecarbonyl, Cyclohexanecarbonyl, Hydrocinnamoyl, trans-Cinnamoyl, Phenylacetyl, Diphenylacetyl, Benzoyl 2-Chlorobenzoyl, 3-Chlorobenzoyl, 4-Chlorobenzoyl, 3,4-Dichlorobenzoyl, 3,5-Dichlorobenzoyl, 2,4-Dichlorobenzoyl, 3,5-Dibromobenzoyl, 4-Fluorobenzoyl, 3-Trifluoromethylbenzoyl, 4-Trifluoromethylbenzoyl, 3-Nitrobenzoyl, 4-Nitrobenzoyl, 3-Dimethylaminobenzoyl, 3-Methoxybenzoyl, 1-Naphthoyl, 2-Naphthoyl, 2-Quinolinecarbonyl, 3-Quinolinecarbonyl, 4-Quinolinecarbonyl, Indole-3-acetyl, Pyrrole-2-carbonyl, 1-Methyl-2-pyrrolecarbonyl, 2-Furoyl, 5-Bromofuroyl, 5-Nitrofuroyl, 3-Thiophenecarbonyl, 2-Thiophenecarbonyl, 2-Thiopheneacetyl, Picolinoyl, Isonicotinoyl, 5,6-Dichloronicotinoyl, 2-Methylnicotinoyl, 6-Methylnicotinoyl, 5-Bromonicotinoyl, 2-Pyrazinecarbonyl, Isobutyryl, Valeryl, Methoxyacetyl or Cyclohexanecarbonyl.

33. Use according to any of claims 24 to 32 wherein the concentration of melatonin present on the stent is between 0.005 and 2 micrograms inclusive melatonin / mm².

34. Use according to any of claims 24 to 33 wherein the concentration of paclitaxel on the stent is between 0.001 and 0.2 micrograms inclusive paclitaxel/mm².

35. Use according to any of claims 24 to 34, wherein said SMC proliferation is restenosis or stenosis.

36. Use according to any of claims 24 to 35 wherein the stent is placed in an artery or vein.

37. Use according to any of claims 24 to 36, wherein the composition comprises melatonin and paclitaxel for separate or simultaneous administration.

38. Use according to claim 37, wherein paclitaxel and melatonin are provided as separate inner and outer layers, one disposed over the other.

39. Use according to claim 38, wherein the inner layer comprises melatonin and the outer layer comprises paclitaxel.

40. Use according to claim 38, wherein the inner layer comprises paclitaxel and the outer layer comprises melatonin.

41. Use according to claim 40, wherein the inner paclitaxel layer and outer melatonin layer are devoid of slow release agents, and said stent is provided with a separate outermost layer comprising at least one slow release agent.

## Patentansprüche

1. Medizinischer Stent, der mit einer Zusammensetzung, die Melatonin und Paclitaxel umfasst, bereitgestellt ist.

2. Medizinischer Stent nach Anspruch 1, wobei der Stent mit einem oder mehreren Hohlräumen ausgestattet ist, die dazu konfiguriert sind, die Zusammensetzung zu enthalten und freizusetzen.

3. Medizinischer Stent nach Anspruch 1 oder 2, wobei der Stent zumindest teilweise aus einem Material hergestellt ist, das *in situ* biologisch abbaubar ist.

4. Medizinischer Stent nach einem der Ansprüche 1 bis 3, wobei der Stent eine Legierung auf Magnesiumbasis umfasst.

5. Medizinischer Stent nach Anspruch 1 oder 3, wobei der Stent zumindest teilweise aus einem Material hergestellt ist, das *in situ* biologisch nicht abbaubar ist.

6. Medizinischer Stent nach einem der Ansprüche 1 bis 5, wobei der Stent zumindest teilweise mit der Zusammensetzung bereitgestellt ist.

7. Medizinischer Stent nach einem der Ansprüche 1 bis 6, wobei die Zusammensetzung ferner ein oder mehrere Mittel zur Freisetzungsverzögerung umfasst, um die verzögerte Freisetzung von Paclitaxel und Melatonin abzustimmen.

8. Medizinischer Stent nach Anspruch 7, wobei das Mittel zur Freisetzungsverzögerung ein beliebiges aus Magnesiumlegierungen, Poly(glykol)säure, Poly(milchsäure) oder im Allgemeinen glykol- und milchsäurebasierten Polymeren, Copolymeren, Polycaprolactonen und im Allgemeinen Polyhydroxylalkanoaten, Poly(hydroxyalkansäuren), Poly(ethylenglykol), Polyvinylalkohol, Poly(orthoestern), Poly(anhydriden), Poly(carbonaten), Polyamiden, Polyimiden, Polyiminen, Poly(iminocarbonaten), Poly(ethyleniminen), Polydioxanen, Polyoxyethylen (Polyethylenoxid), Poly(phosphazenen), Polysulfonen, Lipiden, Polyacrylsäuren, Polymethylmethacrylat, Polyacrylamiden, Polyacrylonitrilen (Polycyanoacrylaten), Poly-HEMA, Polyurethanen, Polyolefinen, Polystyrol, Polyterephthalaten, Polyethylenen, Polypropylenen, Polyetherketonen, Polyvinylchloriden, Polyfluoriden, Siliconen, Polysilicaten (bioaktives Glas), Siloxanen (Polydimethylsiloxanen), Hydroxylapatiten, Lactid-Capronolacton, natürlichen und nicht natürlichen Polyaminosäuren, Poly-β-aminoestern, Albuminen, Alginaten, Cellulose/Celluloseacetaten, Chitin/Chitosan, Collagen, Fibrin/Fibrinogen, Gelatine, Lignin, proteinbasierten Polymeren, Poly(lysin), Poly(glutamat), Poly(malonaten), Poly(hyaluronsäuren), Polynucleinsäuren, Polysacchariden, Poly(hydroxyalkanoaten), Polyisoprenoiden, stärkebasierten Polymeren, Copolymeren davon, linearen, verzweigten, hyperverzweigten, Dendrimeren, vernetzten, funktionalisierten Derivaten davon, oder Hydrogelen auf der Basis aktivierter Polyethylenglykole kombiniert mit alkalisch hydrolysierten tierischen oder pflanzlichen Proteinen ist.

9. Medizinischer Stent nach Anspruch 7, wobei das Mittel zur Freisetzungsverzögerung ein biologisch abbaubares Poly(esteramid)-Copolymer ist.

10. Medizinischer Stent nach einem der Ansprüche 1 bis 9, wobei das Melatonin ein Gemisch aus zumindest einem Melatonin-Analogon, optional gemeinsam mit Melatonin, ist.

11. Medizinischer Stent nach einem der Ansprüche 1 bis 10, wobei das Paclitaxel ein Gemisch aus zumindest einem Paclitaxel-Analogon, optional gemeinsam mit Paclitaxel, ist.

12. Medizinischer Stent nach Anspruch 10 oder 11, wobei ein Melatonin-Analogon ein beliebiges aus 2-Iodomelatonin, 6-Chloromelatonin, 6,7-Dichlor-2-methylmelatonin und 8-Hydroxymelatonin ist.

13. Medizinischer Stent nach Anspruch 11 oder 12, wobei ein Paclitaxel-Analogon eine Verbindung der folgenden Formel (II) ist: 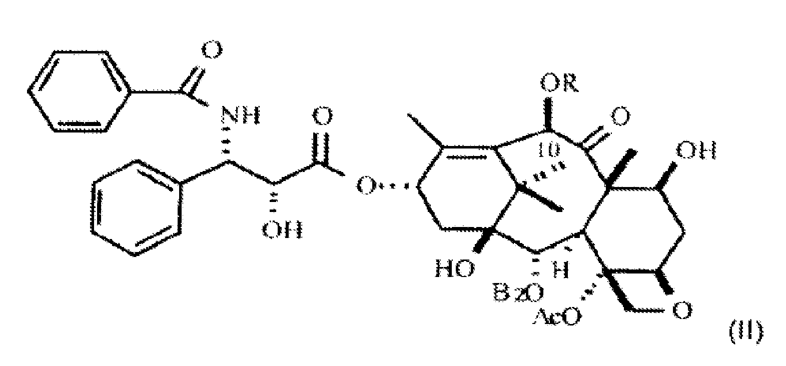 worin R ein beliebiges aus Propionyl, Isobutyryl, Valeryl, Hexanoyl, Octanoyl, Decanoyl, Tridecanoyl, Methoxyacetyl, Methylthioacetyl, Methylsulfonylacetyl, Acetoxyacetyl, Ethylformyl, Monosuccinyl, Crotonoyl, Acryloyl, Cyclopropancarbonyl, Cyclobutancarbonyl, Cyclopentancarbonyl, Cyclohexancarbonyl, Hydrocinnamoyl, trans-Cinnamoyl, Phenylacetyl, Diphenylacetyl, Benzoyl, 2-Chlorbenzoyl, 3-Chlorbenzoyl, 4-Chlorbenzoyl, 3,4-Dichlorbenzoyl, 3,5-Dichlorbenzoyl, 2,4-Dichlorbenzoyl, 3,5-Dibrombenzoyl, 4-Fluorbenzoyl, 3-Trifluormethylbenzoyl, 4-Trifluormethylbenzoyl, 3-Nitrobenzoyl, 4-Nitrobenzoyl, 3-Dimethylaminobenzoyl, 3-Methoxybenzoyl, 1-Naphthoyl, 2-Naphthoyl, 2-Chinolincarbonyl, 3-Chinolincarbonyl, 4-Chinolincarbonyl, Indol-3-acetyl, Pyrrol-2-carbonyl, 1-Methyl-2-pyrrolcarbonyl, 2-Furoyl, 5-Bromfuroyl, 5-Nitrofuroyl, 3-Thiophencarbonyl, 2-Thiophencarbonyl, 2-Thiophenacetyl, Picolinoyl, Isonicotinoyl, 5,6-Dichlornicotinoyl, 2-Methylnicotinoyl, 6-Methylnicotinoyl, 5-Bromnicotinoyl, 2-Pyrazincarbonyl, Isobutyryl, Valeryl, Methoxyacetyl oder Cyclohexancarbonyl ist.

14. Medizinischer Stent nach einem der Ansprüche 1 bis 13, wobei die Melatoninkonzentration, die am Stent gegenwärtig ist, zwischen einschließlich 0,005 und 2 Mikrogramm Melatonin/mm² beträgt.

15. Medizinischer Stent nach einem der Ansprüche 1 bis 14, wobei die Paclitaxelkonzentration am Stent zwischen einschließlich 0,001 und 0,2 Mikrogramm Paclitaxel/mm² beträgt.

16. Medizinischer Stent nach einem der Ansprüche 1 bis 15, der zur Verwendung zur Hemmung der GMZ-Proliferation geeignet ist.

17. Medizinischer Stent nach Anspruch 16, wobei die GMZ-Proliferation eine Restenose oder Stenose ist.

18. Medizinischer Stent nach einem der Ansprüche 1 bis 17, wobei der Stent in einer Arterie oder Vene angeordnet wird.

19. Medizinischer Stent nach einem der Ansprüche 1 bis 18, wobei die Zusammensetzung Melatonin und Paclitaxel zur getrennten oder gleichzeitigen Verabreichung umfasst.

20. Medizinischer Stent nach Anspruch 19, wobei Paclitaxel und Melatonin als separate innere und äußere Schichten, wobei eine über der anderen angeordnet ist, bereitgestellt sind.

21. Medizinischer Stent nach Anspruch 20, wobei die innere Schicht Melatonin umfasst und die äußere Schicht Paclitaxel umfasst.

22. Medizinischer Stent nach Anspruch 20, wobei die innere Schicht Paclitaxel umfasst und die äußere Schicht Melatonin umfasst.

23. Medizinischer Stent nach Anspruch 22, wobei die innere Paclitaxelschicht und die äußere Melatoninschicht frei von Mitteln zur Freisetzungsverzögerung sind und der Stent mit einer äußersten Schicht ausgestattet ist, die ein Mittel zur Freisetzungsverzögerung umfasst.

24. Verwendung einer Zusammensetzung, die Melatonin und Paclitaxel umfasst, zur Herstellung eines Medikaments zur Bereitstellung eines medizinischen Stents zur Behandlung der Proliferation glatter Muskelzellen (GMZ).

25. Verwendung nach Anspruch 24, wobei der Stent so ist, wie er in einem der Ansprüche 2 bis 6 definiert ist.

26. Verwendung nach Anspruch 24 oder 25, wobei die Zusammensatzung ferner ein oder mehrere Mittel zur Freisetzungsverzögerung umfasst, um die verzögerte Freisetzung von Paclitaxel und Melatonin abzustimmen.

27. Verwendung nach Anspruch 26, wobei das Mittel zur Freisetzungsverzögerung ein beliebiges aus Magnesiumlegierungen, Poly(glykol)säure, Poly(milchsäure) oder im Allgemeinen glykol- und milchsäurebasierten Polymeren, Copolymeren, Polycaprolactonen und im Allgemeinen Polyhydroxylalkanoaten, Poly(hydroxyalkansäuren), Poly(ethylenglykol), Polyvinylalkohol, Poly-(orthoestern), Poly(anhydriden), Poly(carbonaten), Polyamiden, Polyimiden, Polyiminen, Poly(iminocarbonaten), Poly(ethyleniminen), Polydioxanen, Polyoxyethylen (Polyethylenoxid), Poly(phosphazenen), Polysulfonen, Lipiden, Polyacrylsäuren, Polymethylmethacrylat, Polyacrylamiden, Polyacrylonitrilen (Polycyanoacrylaten), Poly-HEMA, Polyurethanen, Polyolefinen, Polystyrol, Polyterephthalaten, Polyethylenen, Polypropylenen, Polyetherketonen, Polyvinylchloriden, Polyfluoriden, Siliconen, Polysilicaten (bioaktives Glas), Siloxanen (Polydimethylsiloxanen), Hydroxylapatiten, Lactid-Capronolacton, natürlichen und nicht natürlichen Polyaminosäuren, Poly-β-aminoestern, Albuminen, Alginaten, Cellulose/Celluloseacetaten, Chitin/Chitosan, Collagen, Fibrin/Fibrinogen, Gelatine, Lignin, proteinbasierten Polymeren, Poly(lysin), Poly(glutamat), Poly(malonaten), Poly(hyaluronsäuren), Polynucleinsäuren, Polysacchariden, Poly(hydroxyalkanoaten), Polyisoprenoiden, stärkebasierten Polymeren, Copolymeren davon, linearen, verzweigten, hyperverzweigten, Dendrimeren, vernetzten, funktionalisierten Derivaten davon, oder Hydrogelen auf der Basis aktivierter Polyethylenglykole kombiniert mit alkalisch hydrolysierten tierischen oder pflanzlichen Proteinen ist.

28. Verwendung nach Anspruch 26, wobei das Mittel zur Freisetzungsverzögerung ein biologisch abbaubares Poly(esteramid)-Copolymer ist.

29. Verwendung nach einem der Ansprüche 24 bis 28, wobei das Melatonin ein Gemisch aus zumindest einem Melatonin-Analogon, optional gemeinsam mit Melatonin, ist.

30. Verwendung nach einem der Ansprüche 24 bis 29, wobei das Paclitaxel ein Gemisch aus zumindest einem Paclitaxel-Analogon, optional gemeinsam mit Paclitaxel, ist.

31. Verwendung nach einem der Ansprüche 29 oder 30, wobei ein Melatonin-Analogon ein beliebiges aus 2-Iodomelatonin, 6-Chloromelatonin, 6,7-Dichlor-2-methylmelatonin und 8-Hydroxymelatonin ist.

32. Verwendung nach Anspruch 30 oder 31, wobei ein Paclitaxel-Analogon eine Verbindung der folgenden Formel (II) ist: 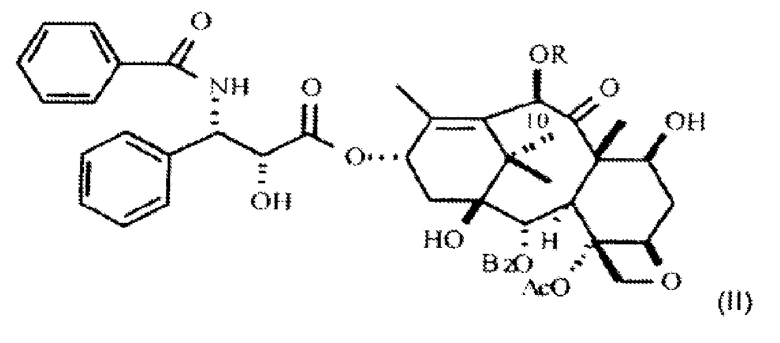 worin R ein beliebiges aus Propionyl, Isobutyryl, Valeryl, Hexanoyl, Octanoyl, Decanoyl, Tridecanoyl, Methoxyacetyl, Methylthioacetyl, Methylsulfonylacetyl, Acetoxyacetyl, Ethylformyl, Monosuccinyl, Crotonoyl, Acryloyl, Cyclopropancarbonyl, Cyclobutancarbonyl, Cyclopentancarbonyl, Cyclohexancarbonyl, Hydrocinnamoyl, trans-Cinnamoyl, Phenylacetyl, Diphenylacetyl, Benzoyl, 2-Chlorbenzoyl, 3-Chlorbenzoyl, 4-Chlorbenzoyl, 3,4-Dichlorbenzoyl, 3,5-Dichlorbenzoyl, 2,4-Dichlorbenzoyl, 3,5-Dibrombenzoyl, 4-Fluorbenzoyl, 3-Trifluormethylbenzoyl, 4-Trifluormethylbenzoyl, 3-Nitrobenzoyl, 4-Nitrobenzoyl, 3-Dimethylaminobenzoyl, 3-Methoxybenzoyl, 1-Naphthoyl, 2-Naphthoyl, 2-Chinolincarbonyl, 3-Chinolincarbonyl, 4-Chinolincarbonyl, Indol-3-acetyl, Pyrrol-2-carbonyl, 1-Methyl-2-pyrrolcarbonyl, 2-Furoyl, 5-Bromfuroyl, 5-Nitrofuroyl, 3-Thiophencarbonyl, 2-Thiophencarbonyl, 2-Thiophenacetyl, Picolinoyl, Isonicotinoyl, 5,6-Dichlornicotinoyl, 2-Methylnicotinoyl, 6-Methylnicotinoyl, 5-Bromnicotinoyl, 2-Pyrazincarbonyl, Isobutyryl, Valeryl, Methoxyacetyl oder Cyclohexancarbonyl ist.

33. Verwendung nach einem der Ansprüche 24 bis 32, wobei die Melatoninkonzentration, die am Stent gegenwärtig ist, zwischen einschließlich 0,005 und 2 Mikrogramm Melatonin/mm² beträgt.

34. Verwendung nach einem der Ansprüche 24 bis 33, wobei die Paclitaxelkonzentration am Stent zwischen einschließlich 0,001 und 0,2 Mikrogramm Paclitaxel/mm² beträgt.

35. Verwendung nach einem der Ansprüche 24 bis 34, wobei die GMZ-Proliferation eine Restenose oder Stenose ist.

36. Verwendung nach einem der Ansprüche 24 bis 35, wobei der Stent in einer Arterie oder Vene angeordnet wird.

37. Verwendung nach einem der Ansprüche 24 bis 36, wobei die Zusammensetzung Melatonin und Paclitaxel zur getrennten oder gleichzeitigen Verabreichung umfasst.

38. Verwendung nach Anspruch 37, wobei Paclitaxel und Melatonin als separate innere und äußere Schichten, wobei eine über der anderen angeordnet ist, bereitgestellt sind.

39. Verwendung nach Anspruch 38, wobei die innere Schicht Melatonin umfasst und die äußere Schicht Paclitaxel umfasst.

40. Verwendung nach Anspruch 39, wobei die innere Schicht Paclitaxel umfasst und die äußere Schicht Melatonin umfasst.

41. Verwendung nach Anspruch 40, wobei die innere Paclitaxelschicht und die äußere Melatoninschicht frei von Mitteln zur Freisetzungsverzögerung sind und der Stent mit einer separaten äußersten Schicht ausgestattet ist, die zumindest ein Mittel zur Freisetzungsverzögerung umfasst.

## Revendications

1. Stent médical contenant une composition comprenant de la mélatonine et du paclitaxel.

2. Stent médical selon la revendication 1, ledit stent comprenant une ou plusieurs cavités configurées pour contenir et libérer ladite composition.

3. Stent médical selon la revendication 1 ou 2, ledit stent étant au moins partiellement formé à partir d'un matériau qui est biodégradable *in situ.*

4. Stent médical selon l'une quelconque des revendications 1 à 3, ledit stent comprenant un alliage à base de magnésium.

5. Stent médical selon la revendication 1 ou 3, ledit stent étant au moins partiellement formé à partir d'un matériau qui est non biodégradable *in situ.*

6. Stent médical selon l'une quelconque des revendications 1 à 5, ledit stent étant au moins partiellement pourvu de ladite composition.

7. Stent médical selon l'une quelconque des revendications 1 à 6, dans lequel ladite composition comprend en outre un ou plusieurs agents à libération prolongée destinés à moduler la libération prolongée du paclitaxel et de la mélatonine.

8. Stent médical selon la revendication 7, dans lequel ledit agent à libération prolongée est l'un quelconque d'alliages de magnésium, de poly(acide glycolique), de poly(acide lactique) ou en général de polymères à base d'acide glycolique et lactique, de copolymères, de poly caprolactones et en général, de poly hydroxyl alcanoates, de poly(acides hydroxy alcanoïques), du poly(éthylène glycol), de l'alcool polyvinylique, de poly(orthoesters), de poly(anhydrides), de poly (carbonates), de polyamides, de polyimides, de polyimines, de poly (imino carbonates), de poly(éthylène imines), de polydioxanes, du poly oxyéthylène (poly oxyde d'éthylène), de poly (phosphazènes), de poly sulfones, de lipides, d'acides poly acryliques, du poly méthacrylate de méthyle, de poly acryl amides, de poly acrylo nitriles (poly cyano acrylates), du poly HEMA, de poly uréthanes, de poly oléfines, du poly styrène, de poly téréphtalates, de poly éthylènes, de poly propylènes, de poly éther cétones, de poly chlorures de vinyle, de poly fluorures, de silicones, de poly silicates (verre bioactif), de siloxanes (poly diméthyl siloxanes), d'hydroxyapatites, du lactide-capronolactone, d'acides poly aminés naturels et non naturels, de poly β-aminoesters, d'albumines, d'alginates, de cellulose/acétates de cellulose, de chitine/chitosane, du collagène, de fibrine/fibrinogène, de la gélatine, de la lignine, de polymères à base de protéines, de la Poly (lysine), du poly (glutamate), de poly (malonates), de poly (acides hyaluroniques), d'acides poly nucléiques, de poly saccharides, de poly (hydroxyalcanoates), de poly isoprénoïdes, de polymères à base d'amidon, de leurs copolymères, linéaires, ramifiés, hyperamifiés, de dendrimères, réticulés, de leurs dérivés fonctionnalisés, ou d'hydrogels à base de polyéthylèneglycols activés combinés à des protéines végétales ou animales alcalines hydrolysées.

9. Stent médical selon la revendication 7, dans lequel ledit agent à libération prolongée est un copolymère biodégradable de poly (ester amide).

10. Stent médical selon l'une quelconque des revendications 1 à 9, dans lequel ladite mélatonine est un mélange d'au moins un analogue de la mélatonine éventuellement conjointement à de la mélatonine.

11. Stent médical selon l'une quelconque des revendications 1 à 10, dans lequel ledit paclitaxel est un mélange d'au moins un analogue du paclitaxel éventuellement conjointement à du paclitaxel.

12. Stent médical selon la revendication 10 ou 11, dans lequel un analogue de la mélatonine est l'un quelconque de la 2-iodomélatonine, de la 6-chloromélatonine, de la 6,7-dichloro-2-méthylmélatonine et de la 8-hydroxymélatonine.

13. Stent médical selon la revendication 11 ou 12, dans lequel un analogue du paclitaxel est un composé de formule (II), 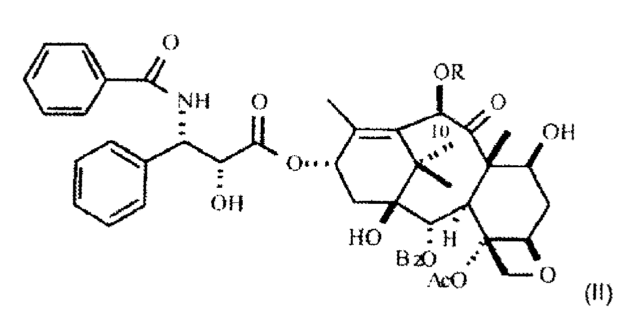 dans laquelle R est l'un quelconque des groupes propionyle, isobutyryle, valéryle, hexanoyle, octanoyle, décanoyle, tridécanoyle, méthoxyacétyle, méthylthioacétyle, méthylsulfonylacetyle, acétoxyacétyle, éthylformyle, monosuccinyle, crotonoyle, acryloyle, cyclopropanecarbonyle, cyclobutanecarbonyle, cyclopentanecarbonyle, cyclohexanecarbonyle, hydrocinnamoyle, transcinnamoyle, phénylacétyle, diphénylacétyle, benzoyle, 2-chlorobenzoyle, 3-chlorobenzoyle, 4-chlorobenzoyle, 3,4-dichlorobenzoyle, 3,5-dichlorobenzoyle, 2,4-dichlorobenzoyle, 3,5-dibromobenzoyle, 4-fluorobenzoyle, 3-trifluorométhylbenzoyle, 4-trifluorométhylbenzoyle, 3-nitrobenzoyle, 4-nitrobenzoyle, 3-diméthylaminobenzoyle, 3-méthoxybenzoyle, 1-naphtoyle, 2-naphtoyle, 2-quinoléinecarbonyle, 3-quinoléinecarbonyle, 4-quinoléinecarbonyle, indole-3-acétyle, pyrrole-2-carbonyle, 1-méthyl-2-pyrrolecarbonyle, 2-furoyle, 5-bromofuroyle, 5-nitrofuroyle, 3-thiophènecarbonyle, 2-thiophènecarbonyle, 2-thiophèneacétyle, picolinoyle, isonicotinoyle, 5,6-dichloronicotinoyle, 2-méthylnicotinoyle, 6-méthylnicotinoyle, 5-bromonicotinoyle, 2-pyrazinecarbonyle, isobutyryle, valéryle, méthoxyacétyle ou cyclohexanecarbonyle.

14. Stent médical selon l'une quelconque des revendications 1 à 13, dans lequel la concentration en mélatonine présente sur le stent est comprise entre 0,005 et 2 microgrammes inclus de mélatonine/mm².

15. Stent médical selon l'une quelconque des revendications 1 à 14, dans lequel la concentration en paclitaxel sur le stent est comprise entre 0,001 et 0,2 microgrammes inclus de paclitaxel/mm².

16. Stent médical selon l'une quelconque des revendications 1 à 15, adapté pour être utilisé dans l'inhibition de la prolifération de SMC.

17. Stent médical selon la revendication 16, dans lequel ladite prolifération des SMC est une resténose ou une sténose.

18. Stent médical selon l'une quelconque des revendications 1 à 17, dans lequel le stent est placé dans une artère ou dans une veine.

19. Stent médical selon l'une quelconque des revendications 1 à 18, dans lequel la composition comprend de la mélatonine et du paclitaxel pour une administration séparée ou simultanée.

20. Stent médical selon la revendication 19, dans lequel le paclitaxel et la mélatonine sont fournis sous la forme de couches interne et externe distinctes, l'une sur l'autre.

21. Stent médical selon la revendication 20, dans lequel la couche interne comprend de la mélatonine et la couche externe comprend du paclitaxel.

22. Stent médical selon la revendication 20, dans lequel la couche interne comprend du paclitaxel et la couche externe comprend de la mélatonine.

23. Stent médical selon la revendication 22, dans lequel la couche interne de paclitaxel et la couche externe de mélatonine ne comprennent pas d'agent à libération prolongée, et ledit stent est doté d'une couche extérieure comprenant un agent à libération prolongée.

24. Utilisation d'une composition comprenant de la mélatonine et du paclitaxel, pour la préparation d'un médicament destiné à fournir un stent médical pour le traitement de la prolifération des cellules des muscles lisses, SMC.

25. Utilisation selon la revendication 24, dans laquelle ledit stent est tel que défini dans l'une quelconque des revendications 2 à 6.

26. Utilisation selon la revendication 24 ou 25, dans laquelle ladite composition comprend en outre un ou plusieurs agents à libération prolongée destinés à moduler la libération prolongée du paclitaxel et de la mélatonine.

27. Utilisation selon la revendication 26, dans laquelle ledit agent à libération prolongé est l'un quelconque d'alliages de magnésium, de poly(acide glycolique), de poly(acide lactique) ou en général de polymères à base d'acide glycolique et lactique, de copolymères, de poly caprolactones et en général, de poly hydroxyl alcanoates, de poly(acides hydroxy alcanoïques), du poly(éthylène glycol), de l'alcool polyvinylique, de poly(orthoesters), de poly(anhydrides), de poly (carbonates), de polyamides, de polyimides, de polyimines, de poly (imino carbonates), de poly(éthylène imines), de polydioxanes, du poly oxyéthylène (poly oxyde d'éthylène), de poly (phosphazènes), de poly sulfones, de lipides, d'acides poly acryliques, du poly méthacrylate de méthyle, de poly acryl amides, de poly acrylo nitriles (poly cyano acrylates), du poly HEMA, de poly uréthanes, de poly oléfines, du poly styrène, de poly téréphtalates, de poly éthylènes, de poly propylènes, de poly éther cétones, de poly chlorures de vinyle, de poly fluorures, de silicones, de poly silicates (verre bioactif), de siloxanes (poly diméthyl siloxanes), d'hydroxyapatites, du lactide-capronolactone, d'acides poly aminés naturels et non naturels, de poly β-aminoesters, d'albumines, d'alginates, de cellulose/acétates de cellulose, de chitine/chitosane, du collagène, de fibrine/fibrinogène, de la gélatine, de la lignine, de polymères à base de protéines, de la Poly (lysine), du poly (glutamate), de poly (malonates), de poly (acides hyaluroniques), d'acides poly nucléiques, de poly saccharides, de poly (hydroxyalcanoates), de poly isoprénoïdes, de polymères à base d'amidon, de leurs copolymères, linéaires, ramifiés, hyperamifiés, de dendrimères, réticulés, de leurs dérivés fonctionnalisés, ou d'hydrogels à base de polyéthylèneglycols activés combinés à des protéines végétales ou animales alcalines hydrolysées.

28. Utilisation selon la revendication 26, dans laquelle ledit agent à libération prolongée est un copolymère biodégradable de poly (ester amide).

29. Utilisation selon l'une quelconque des revendications 24 à 28, dans laquelle ladite mélatonine est un mélange d'au moins un analogue de la mélatonine éventuellement conjointement à de la mélatonine.

30. Utilisation selon l'une quelconque des revendications 24 à 29, dans laquelle ledit paclitaxel est un mélange d'au moins un analogue du paclitaxel éventuellement conjointement à du paclitaxel.

31. Utilisation selon l'une quelconque des revendications 29 ou 30, dans laquelle un analogue de la mélatonine est l'un quelconque de la 2-iodomélatonine, de la 6-chloromélatonine, de la 6,7-dichloro-2-méthylmélatonine et de la 8-hydroxymélatonine.

32. Utilisation selon la revendication 30 ou 21, dans laquelle un analogue du paclitaxel est un composé de formule (II), 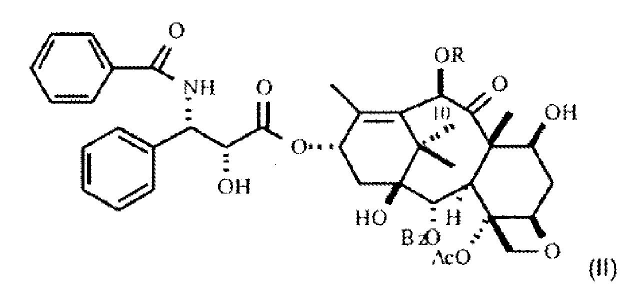 dans laquelle R est l'un quelconque des groupes propionyle, isobutyryle, valéryle, hexanoyle, octanoyle, décanoyle, tridécanoyle, méthoxyacétyle, méthylthioacétyle, méthylsulfonylacetyle, acétoxyacétyle, éthylformyle, monosuccinyle, crotonoyle, acryloyle, cyclopropanecarbonyle, cyclobutanecarbonyle, cyclopentanecarbonyle, cyclohexanecarbonyle, hydrocinnamoyle, transcinnamoyle, phénylacétyle, diphénylacétyle, benzoyle, 2-chlorobenzoyle, 3-chlorobenzoyle, 4-chlorobenzoyle, 3,4-dichlorobenzoyle, 3,5-dichlorobenzoyle, 2,4-dichlorobenzoyle, 3,5-dibromobenzoyle, 4-fluorobenzoyle, 3-trifluorométhylbenzoyle, 4-trifluorométhylbenzoyle, 3-nitrobenzoyle, 4-nitrobenzoyle, 3-diméthylaminobenzoyle, 3-méthoxybenzoyle, 1-naphtoyle, 2-naphtoyle, 2-quinoléinecarbonyle, 3-quinoléinecarbonyle, 4-quinoléinecarbonyle, indole-3-acétyle, pyrrole-2-carbonyle, 1-méthyl-2-pyrrolecarbonyle, 2-furoyle, 5-bromofuroyle, 5-nitrofuroyle, 3-thiophènecarbonyle, 2-thiophènecarbonyle, 2-thiophèneacétyle, picolinoyle, isonicotinoyle, 5,6-dichloronicotinoyle, 2-méthylnicotinoyle, 6-méthylnicotinoyle, 5-bromonicotinoyle, 2-pyrazinecarbonyle, isobutyryle, valéryle, méthoxyacétyle ou cyclohexanecarbonyle.

33. Utilisation selon l'une quelconque des revendications 24 à 32, dans laquelle la concentration en mélatonine présente sur le stent est comprise entre 0,005 et 2 microgrammes inclus de mélatonine/mm².

34. Utilisation selon l'une quelconque des revendications 24 à 33, dans laquelle la concentration en paclitaxel sur le stent est comprise entre 0,001 et 0,2 microgrammes inclus de paclitaxel/mm².

35. Utilisation selon l'une quelconque des revendications 24 à 34, dans laquelle ladite prolifération des SMC est une resténose ou une sténose.

36. Utilisation selon l'une quelconque des revendications 24 à 35, dans laquelle le stent est placé dans une artère ou dans une veine.

37. Utilisation selon l'une quelconque des revendications 24 à 36, dans laquelle la composition comprend de la mélatonine et du paclitaxel pour une administration séparée ou simultanée.

38. Utilisation selon la revendication 37, dans laquelle le paclitaxel et la mélatonine sont fournis sous la forme de couches interne et externe distinctes, l'une sur l'autre.

39. Utilisation selon la revendication 38, dans laquelle la couche interne comprend de la mélatonine et la couche externe comprend du paclitaxel.

40. Utilisation selon la revendication 38, dans laquelle la couche interne comprend du paclitaxel et la couche externe comprend de la mélatonine.

41. Utilisation selon la revendication 40, dans laquelle la couche interne de paclitaxel et la couche externe de mélatonine ne comprennent pas d'agent à libération prolongée, et ledit stent est doté d'une couche extérieure distincte comprenant au moins un agent à libération prolongée.
